(19) Europäisches Patentamt
European Patent Office
Office européen des brevets

(11) Veröffentlichungsnummer: **0 004 952**
**B1**

# (12) EUROPÄISCHE PATENTSCHRIFT

(45) Veröffentlichungstag der Patentschrift: **07.04.82**

(21) Anmeldenummer: **79101135.6**

(22) Anmeldetag: **12.04.79**

(51) Int. Cl.³: **C 07 D 491/107,**
A 61 K 31/40,
A 61 K 31/445
//(C07D491/107, 207/12,
211/46, 307/00)

(54) **Spiro-(dihydrobenzofuranpiperidine und -pyrrolidine) und deren Derivate, Verfahren zu ihrer Herstellung und ihre Verwendung für Arzneimittel.**

(30) Priorität: **14.04.78 US 896622**

(43) Veröffentlichungstag der Anmeldung:
**31.10.79 Patentblatt 79/22**

(45) Bekanntmachung des Hinweises auf die Patenterteilung:
**07.04.82 Patentblatt 82/14**

(84) Benannte Vertragsstaaten:
**DE FR GB IT**

(56) Entgegenhaltungen:
**DE - A - 2 458 176**
**DE - A - 2 458 177**
**US - A - 3 686 186**
**US - A - 2 500 714**

**Journal of Medicinal Chemistry**
**Band 19, Nr. 11,1976**
**Washington, USA**
**V. J. BAUER et al. "Synthesis of spiro [isobenzofuran-1(3H),4'-piperidines] as potential central nervous system agents. 1"**
**Seiten 1315 bis 1324**

(73) Patentinhaber: **HOECHST AKTIENGESELLSCHAFT**
**Postfach 80 03 20**
**D-6230 Frankfurt/Main 80 (DE)**

(72) Erfinder: **Effland, Richard Charles**
**Rolling Hills Road 544**
**Brigdewater, N.J. (US)**
Erfinder: **Strupczewski, Joseph Thomas**
**Garden Lane 8**
**Flemington, N.J. (US)**
Erfinder: **Gardner, Beth Ann**
**Calcaterra Drive 7038**
**San José, California (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Spiro[dihydrobenzofuranpiperidine und -pyrrolidine] und deren Derivate, Verfahren zu ihrer Herstellung und ihre Verwendung für Arzneimittel

Gegenstand der vorliegenden Erfindung sind neue Spiro[dihydrobenzofuranpiperidine und -pyrrolidine] sowie deren pharmazeutisch unbedenkliche Säureadditionssalze, welche wertvolle Analgetika, Beruhigungsmittel und Zwischenprodukte für deren Herstellung darstellen, Verfahren zu ihrer Herstellung sowie Arzneimittel, die diese Verbindung als Hauptwirkstoffe enthalten.

Die erfindungsgemäßen Verbindungen haben die Formel

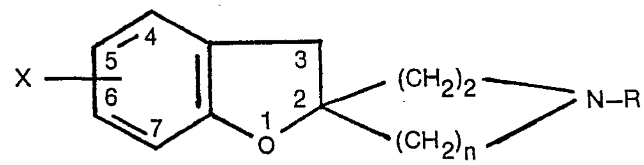

I

worin

X Wasserstoff, Nitro, Amino, Chlor, Fluor, Brom oder Methoxy,

R Wasserstoff, $C_{1-6}$-Alkyl, $C_{2-6}$-Alkenyl, Hydroxyäthyl, $C_{1-6}$-Alkanoyl, $C_{1-6}$-Alkoxyoxalyl, unsubstituiertes oder substituiertes Phenyl-$C_{1-6}$-alkanoyl, Benzoyl, Phenyl-$C_{1-6}$-alkyl oder Phenoxy-$C_{1-6}$-alkyl, wobei der Phenylrest jeweils ein oder zweifach mit Nitro, Amino-, Chlor, Fluor, Brom oder Methoxy substituiert sein kann, $C_{3-7}$-Cycloalkyl-$C_{1-6}$-alkyl, $C_{3-7}$-Cycloalkylcarbonyl, $C_{1-6}$-Alkoxycarbonyl, Phenoxycarbonyl, Furfuryl, Furoyl, N-[2-(3-indolyläthyl] oder N-(indol-3-yl-oxalyl),

n 1 oder 2

bedeuten.

Falls nicht anders angegeben, bedeuten die Bezeichnungen "Alkyl", "Alkenyl", "Alkanoyl" und "Alkoxy" — allein oder zusammen mit anderen Bezeichnungen wie "Phenyl" oder "Cycloalkyl" — solche Gruppen, die bis zu 6 vorzugsweise bis zu 4 Kohlenstoffatome aufweisen; "Cycloalkyl" bedeutet Cycloalkyl mit 3 bis 7, vorzugsweise 4 bis 6 Kohlenstoffatomen, und die Bezeichnung "substituiert" — im Hinblick auf "Phenylalkanoyl", "Benzoyl", "Phenylalkyl" oder "Phenoxyalkyl" — steht für einen oder mehrere, vorzugsweise 1 oder 2 Nitro-, Amino-, Chlor-, Fluor-, Brom- oder Methoxy-Substituenten.

Als geeignete Säuren für die Herstellung der pharmazeutisch unbedenklichen sauren Salze gemäß der Erfindung kommen anorganische Säuren wie Salzsäure, Bromwasserstoffsäure, Schwefelsäure, Salpetersäure, Phosphorsäure und Perchlorsäuren sowie organische Säuren, beispielsweise Weinsäure, Zitronensäure, Essigsäure, Bernsteinsäure, Maleinsäure und Fumarsäure in Frage.

Die erfindungsgemäßen Verbindungen sind bisher noch nicht bekannt. Bisher wurden Spiro-[phthalanpiperidine] der Formel

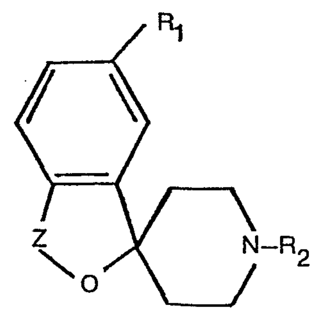

in welcher

$R_1$ Wasserstoff, niederes Alkyl, niederes Alkoxy, Halogen oder Trifluormethyl,

$R_2$ Wasserstoff oder Benzyl und

Z $-CH_2-$ oder $-CO-$ bedeuten von W. J. Houlihan et al. in U.S. Patent No. 3 686 186 beschrieben, ferner die natürliche Verbindung der Formel

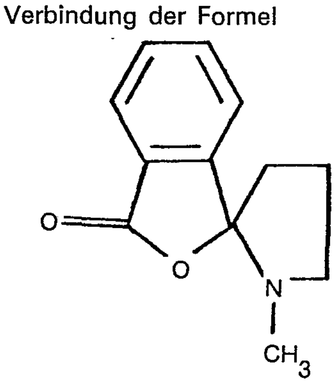

von Y. Inubushi et al. [Chem. and Pharm. Bull (Japan) *12*, 749, (1964)], sowie substituierte 1,3-Dihydrospiro-(isobenzofurane) der Formel

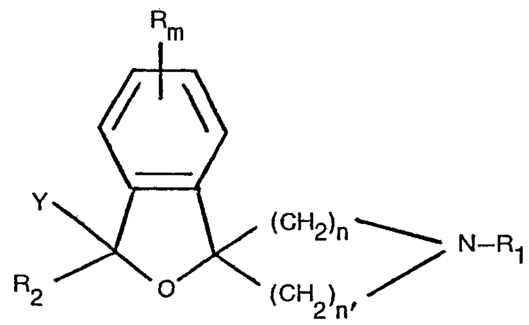

worin
R Wasserstoff, Alkyl, Alkoxy, Trifluormethyl, Halogen, Hydroxy oder Methylendioxy,
$R_1$ Wasserstoff, Alkyl, Cycloalkylalkyl, Alkenyl, Phenylalkyl, Diphenylalkyl, Diphenylmethoxyalkyl, Alkanoyl, Phenylalkanoyl, Benzoyl, Benzoylalkyl, Phenylhydroxyalkyl, Alkoxycarbonyl, Phenyloxycarbonyl oder Cycloalkylcarbonyl,
$R_2$ Alkyl oder Phenyl,
Y Wasserstoff, Alkyl, Alkoxy, Hydroxy oder Phenyl und
m, n und n' die Zahlen 1 bis 3 bedeuten,
und 1,3-Dihydrospiro-(isobenzofurane) der Formel

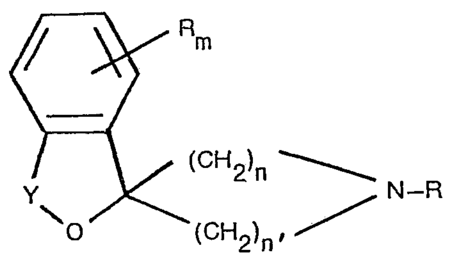

in welcher
R Wasserstoff, Alkyl, Alkoxy, Trifluormethyl, Halogen, Hydroxy oder Methylendioxy,
$R_1$ Alkyl, Cycloalkylalkyl, Phenylalkyl, Diphenylalkyl, Diphenylmethoxyalkyl, Alkanoyl, Phenylalkanoyl, Benzoylalkyl, Phenylhydroxyalkyl oder Cycloalkylcarbonyl,
Y $CH_2$ oder CO,
*m* 1 oder 2 und
*n* und *n'* ganze Zahlen von 1 bis 3 bedeuten,
von Victor J. Bauer und Raymond W. Kosley, Jr. in den U.S. Patenten 3 959 475 bzw. 3 962 259.

Die Herstellung der erfindungsgemäßen Verbindungen erfolgt gemäß den nachfolgend beschriebenen Reaktionsschritten. Falls nicht anders angegeben, haben X, R und n dieselbe Bedeutung wie oben angegeben.

Ein 2-Fluorbenzylchlorid oder -bromid der Formel

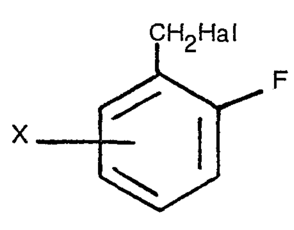

II

worin Hal Chlor oder Brom bedeutet, wird in sein Grignard-Reagenz überführt, vorzugsweise unter Verwendung von Äther als Lösungsmittel und einem Jodkristall als Reaktionsinitiator. Das Grignard-Reagenz wird sodann mit einem Cycloazalkanon der Formel

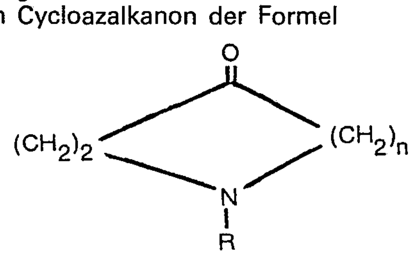

III

worin R für $C_{1-6}$-Alkyl, $C_{2-6}$-Alkenyl, $C_{3-7}$-Cycloalkyl-$C_{1-6}$-alkyl oder Phenyl-$C_{1-6}$-alkyl stehen kann, zu Verbindungen der Formel

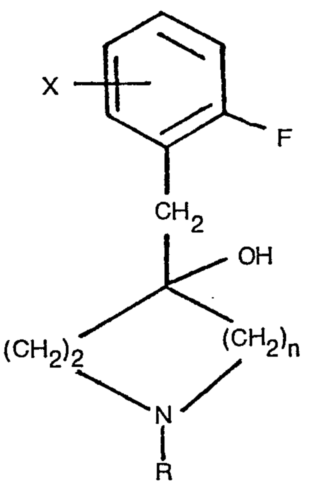

IV

umgesetzt, welche direkte Vorprodukte der erfindungsgemäßen Verbindungen sind. Verbindungen, die in diese Gruppe von Vorprodukten fallen, sind bereits in "Fluorocompounds Related to the Reversed Esters of Pethidine" von N. J. Harper und A. B. Simonds Journal of Medicinal and Pharmaceutical Chemistry, Bd. 1, Nr. 2 (1959) beschrieben worden.

Diese Vorprodukte der Formel IV werden mit einer nicht nucleophilen Base in Gegenwart eines Lösungsmittels bei Temperaturen von 25°C bis zur Rückflußtemperatur des Lösungsmittels zu erfindungsgemäßen Verbindungen umgesetzt, nämlich N-substituierten Spiro[dihydrobenzofuran-piperidinen] oder N-substituierten Spiro[dihydrobenzofuranpyrrolidinen] der Formel

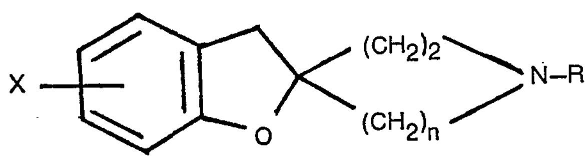

I

worin R $C_{1-6}$-Alkyl, $C_{2-6}$-Alkenyl, $C_{3-7}$-Cycloalkyl-$C_{1-6}$-alkyl oder Phenyl-$C_{1-6}$-alkyl bedeutet. Gemäß einer bevorzugten Ausführungsform werden Natriumhydrid als Base und Dimethylformamid und Benzol als Lösungsmittelgemisch bei Rückflußtemperatur eingesetzt.

Eine so hergestellte N-Benzyl-Verbindung der Formel I, worin R Benzyl ist, läßt sich nach geeigneten Methoden zur entsprechenden N-unsubstituierten Verbindung der Formel I, worin R = H ist hydrieren. Eine bevorzugte Methode stellt die Hydrierung mit einem Palladium-Kohle-Katalysator dar.

Nach einer anderen Methode zur Herstellung der N-unsubstituierten erfindungsgemäßen Verbindungen kann man eine N-substituierte Verbindung der Formel I, in welcher R $C_{1-6}$-Alkyl, $C_{2-6}$-Alkenyl, $C_{3-7}$-Cycloalkyl-$C_{1-6}$-alkyl oder Phenyl-$C_{1-6}$-alkyl bedeutet, mit einem Chlorameisensäureester, wie beispielsweise einem Chlorameisensäure-$C_{1-6}$-alkyl- oder -phenylester, bei einer Temperatur von 25—125°C in einem Lösungsmittel wie Toluol oder Benzol zur entsprechenden N-$C_{1-6}$-Alkoxycarbonyl- oder N-Phenoxycarbonyl-Verbindung der Formel I, worin R = $CO_2$-$C_{1-6}$-Alkyl oder $CO_2$-Phenyl ist, umsetzen. Die erhaltene Verbindung kann wiederum unter Rückflußbedingungen mit einer Base wie Natrium- oder Kaliumhydroxid in einem Lösungsmittel wie Wasser oder Äthanol oder mit einer Säure wie Bromwasserstoff in Essigsäure zur entsprechenden N-unsubstituierten Verbindung der Formel I mit R = H umgesetzt werden.

Eine N-unsubstituierte Verbindung der Erfindung kann in bekannter Weise mit einem $C_{1-6}$-Alkanoylhalogenid oder -anhydrid, einem $C_{3-7}$-Cycloalkylcarbonylhalogenid, Phenyl-$C_{1-6}$-alkanoylhalogenid, Benzoylhalogenid oder -anhydrid, Furoylhalogenid oder -anhydrid oder Äthyloxalylhalogenid zur entsprechenden N-substituierten Verbindung gemäß der Erfindung umgesetzt werden, in welcher R $C_{1-6}$-Alkanoyl, $C_{3-7}$-Cycloalkylcarbonyl, Phenyl-$C_{1-6}$-alkanoyl, Benzoyl, Furoyl oder Äthyloxalyl bedeutet. In dieser Reaktion verwendet man ein Lösungsmittel wie Pyridin oder Chloroform, und gegebenenfalls einen Säurefänger wie Natriumbicarbonat, Kaliumbicarbonat oder Triäthylamin. Die Reaktionstemperatur kann von ca. 0°C bis zur Rückflußtemperatur des Lösungsmittels variieren, wobei unter Rückflußbedingungen gewöhnlich die Umsatzgeschwindigkeit gesteigert wird.

Eine auf diese Weise hergestellte N-substituierte Verbindung kann zur entsprechenden erfindungsgemäßen Verbindung der Formel I, in welcher R $C_{1-6}$-Alkyl, $C_{3-7}$-Cycloalkyl-$C_{1-6}$-alkyl, Phenyl-$C_{1-6}$-alkyl oder Furfuryl bedeutet, reduziert werden. Bevorzugte Reduktionsmittel für diesen Schritt sind Diboran, Lithium-Aluminium-Hydrid oder Natrium-bis-(2-methoxyäthoxy)-aluminium-

hydrid (VITRIDE[R]) in einem Lösungsmittel wie Tetrahydrofuran oder Benzol.

In ähnlicher Weise kann eine N-$C_{1-6}$-Alkoxyoxalyl-Verbindung der Formel I, die wie oben beschrieben hergestellt wurde, zur entsprechenden Verbindung mit R = Hydroxyäthyl reduziert werden.

Eine N-unsubstituierte Verbindung der Formel I läßt sich mit einer Verbindung, die durch die Formel R—Y dargestellt werden kann, in welcher R $C_{1-6}$-Alkyl, $C_{2-6}$-Alkenyl, Phenyl-$C_{1-6}$-alkyl, Furfuryl, Indol-3-yloxalyl oder

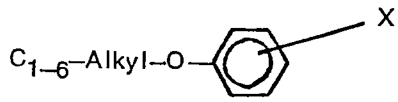

und Y Halogen, vorzugsweise Chlor oder Brom, bedeuten, zur entsprechenden N-substituierten Verbindung der Formel I umsetzen, in welcher R die oben angegebene Bedeutung hat. Dabei wird die Reaktion in einem Lösungsmittel, z.B. Isopropylalkohol oder Dimethylformamid, bei Raumtemperatur bis zur Rückflußtemperatur des Reaktionsgemisches durchgeführt. Man kann gegebenenfalls einen Säurefänger wie Kaliumcarbonat oder Triäthylamin und einen Reaktionsauslöser wie Kaliumjodid einsetzen.

Eine so hergestellte N-(Indol-3-ylglyoxyloyl)-Verbindung wird unter Verwendung von Lithium-Aluminium-Hydrid gemäß der vorbeschriebenen Methode zur entsprechenden N-[2-(3-Indolyl)äthyl]-Verbindung der Formel I reduziert, in welcher R den Rest

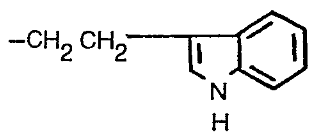

darstellt.

Eine erfindungsgemäße Verbindung, in der X Wasserstoff und R $C_{1-6}$-Alkyl oder $C_{1-6}$-Alkanoyl bedeuten und die nach einer der oben angegebenen Methoden hergestellt wurde, läßt sich mit einer Mischung aus konzentrierter Salpetersäure und Eisessig zur entsprechenden Verbindung der Formel I umsetzen, die in 5-Stellung der Ringstruktur durch eine Nitro-Gruppe substituiert ist. Diese Reaktion wird bei einer Temperature von 25 bis 150°C, vorzugsweise 100°C, durchgeführt.

Ferner kann man eine Verbindung der Formel I, in der R $C_{1-6}$-Alkanoyl bedeutet, zur entsprechenden N-unsubstituierten Verbindung der Formel I, worin R = H ist, hydrolysieren, z.B. mit Hilfe einer starken Säure wie 6n-Salzsäure unter Rückflußbedingungen.

Schließlich kann eine Verbindung der Formel I, in welcher X $NO_2$ bedeutet, zur entsprechenden Verbindung, worin X Amino ist, katalytisch reduziert werden, z.B. durch Hydrierung unter erhöhtem Druck unter Verwendung eines Raney-Nickel-Katalysators.

Die Verbindungen gemäß der vorliegenden Erfindung sind wertvolle Analgetika auf Grund ihrer Fähigkeit, Schmerzen bei Säugetieren zu lindern. Diese analgetische Wirksamkeit der genannten Verbindungen läßt sich mit dem Phenyl-2-chinon-Strecktest an Mäusen, einem Standardtest für Analgetica [Proc. Soc. Exptl. Biol. Med. *95*, 729 (1957)] nachweisen. In nachfolgender Tabelle ist die analgetische Wirkung bei subkutaner Verabreichung verschiedener Dosen typischer Vertreter der erfindungsgemäßen Verbindungen als Prozentsatz der Tiere aufgeführt, bei denen das "Sichkrümmen" aufgrund von Schmerzen unterdrückt wird.

| Verbindung | Dosis mg/kg | Hemmung in Prozent |
|---|---|---|
| 2,3-Dihydro-1′-methylspiro-[benzofuran-2,4′-piperidin]-hydrochlorid | 10 | 38 |
| 2,3-Dihydro-1′-cyclobutylmethylspiro-[benzofuran-2,4′-piperidin]-hydrochlorid | 1,6 | 50 |
| 2,3-Dihydro-1′-(β-phenäthyl)spiro-[benzofuran-2,4′-piperidin]-hydrochlorid | 3,3 | 50 |
| 2,3-Dihydro-1′-(β-phenäthyl)spiro-[benzofuran-2,3′-pyrrolidin]-oxalat | 9,2 | 50 |
| 2,3-Dihydro-1′-5-nitrospiro-[benzofuran-2,4′-piperidin]-hydrochlorid | 1,0 | 68 |

Im Vergleich dazu erzielt man mit den bekannten Analgetika Aspirin und Propoxyphen mit einer Dosis von 60 mg/kg bzw. 28 mg/kg eine Hemmung von 34% bzw. 50%. Daraus geht hervor, daß die Verbindungen gemäß der Erfindung geeignete schmerzlindernde Mittel für Säugetiere sind, wenn sie in

Mengen von 0,05 bis ca. 100 mg/kg Körpergewicht pro Tag verabreicht werden.

Die erfindungsgemäßen Verbindungen sind hinsichtlich der analgetischen Wirksamkeit unerwarteterweise auch den 1,3-Dihydrospiro[isobenzofuran]-Derivaten, die in der DE—A 2 458 176 (entspr. dem oben genannten U.S. Patent 3 962 259) beschrieben sind, überlegen. Von diesen Verbindungen sind Dosen von 17,5 bzw. 50 mg/kg notwendig, um eine Schmerz-Hemmung von 50 bzw. 68% erzielen.

Die Verbindungen der vorliegenden Erfindung eignen sich ebenfalls als Tranquilizer aufgrund ihrer Fähigkeit, beruhigend auf das zentrale Nervensystem von Säugetieren einzuwirken, z.B. bei Verabreichung in Tagesdosen von 0,05 bis ca. 100 mg/kg.

Weitere erfindungsgemäße Verbindungen sind unter anderem:
2,3-Dihydro-5-aminospiro[benzofuran-2,4'-piperidin];
2,3-Dihydro-1'-cyclohexylmethylspiro[benzofuran-2,4'-piperidin];
2,3-Dihydro-5-methoxyspiro[benzofuran-2,4'-piperidin];
2,3-Dihydro-5-methoxyspiro[benzofuran-2,3'-pyrrolidin];
2,3-Dihydro-5-fluor-1'n-propylspiro[benzofuran-2,4'-piperidin];
2,3-Dihydro-6-methoxyspiro[benzofuran-2,3'-pyrrolidin];
2,3-Dihydro-1'-äthylspiro[benzofuran-2,3'-pyrrolidin];
2,3-Dihydro-1'-(trimethoxybenzoyl)spiro[benzofuran-2,3'-pyrrolidin];
2,3-Dihydro-6-bromspiro[benzofuran-2,3'-pyrrolidin];
2,3-Dihydro-1'-benzoylspiro[benzofuran-2,4'-piperidin];
2,3-Dihydro-7-nitrospiro[benzofuran-2,4'-piperidin];
2,3-Dihydro-4-chlorspiro[benzofuran-2,4'-piperidin]; und
2,3-Dihydro-1'-phenoxycarbonylspiro[benzofuran-2,4'-piperidin];

Wirksame Mengen der erfindungsgemäßen Verbindungen können einem Patienten nach den verschiedensten Methoden, beispielsweise oral in Kapseln- oder Tablettenform, parenteral in Form von sterilen Lösungen oder Suspensionen und in einigen Fällen intravenös in Form von sterilen Lösungen, verabreicht werden. Die Endprodukte selbst sind zwar als frei Basen wirksam, doch kann man sie aus Gründen der Stabilität, einer besseren Kristallisierbarkeit, einer erhöhten Löslichkeit usw. in Form ihrer pharmazeutisch unbedenklichen Additionssalze formulieren und verabreichen.

Die erfindungsgemäßen Wirkstoffe können oral verabreicht werden, z.B. mit einem inerten Verdünnungsmittel oder mit einem eßbaren Träger, in Gelatinekapseln eingeschlossen werden oder zu Tabletten verpreßt werden. Zur peroralen therapeutischen Verabreichung können die erfindungsgemäßen Wirkstoffe in Trägerstoffe eingearbeitet werden und in Form von Tabletten, Pastillen, Kapseln, Elixieren, Suspensionen, Sirupen, Oblaten, Kaugummi und dergleichen verabreicht werden. Diese Präparate sollten mindestens 0,5% Wirkstoff enthalten, doch läßt sich dies in Abhängigkeit von der jeweiligen Form variieren. Zweckmäßigerweise beträgt der Wirkstoffgehalt 4 bis ca. 70% des Einheitsgewichts. Die Wirkstoffmenge sollte so bemessen sein, daß sich eine geeignete Dosis ergibt. Bevorzugte Mittel und Präparate gemäß vorliegender Erfindung werden so zubereitet, daß eine perorale Einzeldosis 1,0—300 mg Wirkstoff enthält.

Die Tabletten, Pillen, Kapseln, Pastillen usw. können ferner folgende Bestandteile enthalten: Bindemittel wie mikrokristalline Cellulose, Traganthgummi oder Gelatine; Trägerstoffe wie Stärke oder Milchzucker; Zerfallmittel wie Alginsäure oder Maisstärke usw.; Schmiermittel wie Magnesiumstearat; Gleitmittel wie kolloides Siliziumdioxid und Süßstoffe wie Rohrzucker oder Saccharin oder Aromastoffe wie Pfefferminz, Methylsalicylat oder Orangenaroma. Ist die Dosiereinheit eine Kapsel, so kann diese neben obigen Stoffen flüssige Träger wie pflanzliche Öle enthalten. Andere Dosiereinheiten können weitere Stoffe enthalten, die die physikalische Form der Dosiereinheit ändern, z.B. Überzüge. So können Tabletten und Pillen mit Zucker, Schellack oder anderen erst im Darm löslichen Überzügen versehen werden. Ein Sirup kann neben den Wirkstoffen Rohrzucker als Süßstoff und gewisse Konservierungsmittel, Farbstoffe und Aromastoffe enthalten. Die bei der Zubereitung dieser verschiedenen Mittel verwendeten Stoffe müssen pharmazeutisch rein und nichttoxisch in den angewendeten Mengen sein.

Zur parenteralen therapeutischen Verabreichung können die erfindungsgemäßen Wirkstoffe in eine Lösung oder Suspension eingearbeitet werden. Die so erhaltenen Präparate sollten mindestens 0,1% Wirkstoff enthalten, doch läßt sich dies zwischen 0,5 und ca. 30%, bezogen auf ihr Gewicht, variieren. Die Wirkstoffmenge in diesen Mitteln sollte so bemessen sein, daß eine geeignete Dosis erhalten wird. Bevorzugte Mittel und Präparate gemäß vorliegender Erfindung werden so hergestellt, daß eine parenterale Einzeldosis 0,5—100 mg Wirkstoff enthält.

Die Lösungen oder Suspensionen können ferner folgende Bestandteile enthalten: ein steriles Verdünnungsmittel wie Aqua pro injectione, eine physikalische Kochsalzlösung, nichtflüchtige Öle, Polyäthylenglykole, Glycerin, Propylenglykol oder andere synthetische Lösungsmittel; antibakteriell wirksame Mittel wie Benzylalkohol oder Methylparabene; Antioxidantien wie Ascorbinsäure oder Natriumbisulfit; Chelatbildner wie Äthylendiamintetraessigsäure; Puffersubstanzen wie Acetate, Citrate oder Phosphate und Mittel zur Einstellung des osmotischen Druckes wie Kochsalz oder Dextrose. Die parenterale Zubereitung kann in Ampullen, Einwegspritzen oder Mehrfachdosisspritzen aus Glas oder Plastik eingeschmolzen werden.

In den nachfolgenden Beispielen wird die Erfindung näher erläutert:

Beispiel 1

a) Ein Gemisch aus 2,7 g Magnesiumspänen in 25 ml Äther, das einen Jodkristall enthält, wird mit einigen Millilitern 2-Fluorbenzylchlorid (in einer Lösung aus 14,5 g 2-Fluorbenzylchlorid in 75 ml Äther) versetzt. Die Reaktion wird durch vorsichtiges Erwärmen mit heißer Luft in Gang gesetzt. Nach Beginn der Reaktion tropft man die restliche 2-Fluorbenzylchlorid-Lösung zu, wobei das Reaktionsgemisch auf Rückflußtemperatur erhitzt wird. Nach beendeter Zugabe rührt man das Reaktionsgemisch unter Rückfluß weitere 15 Minuten und tropft dann unter starkem Rühren eine Lösung von N-Benzyl-4-piperidon in 75 ml Äther zu. Die erhaltene Suspension wird bei Zimmertemperatur etwa 2 Stunden lang gerührt und dann filtriert. Der Filterkuchen wird sorgfältig mit Äther gewaschen und anschließend durch Rühren mit einer Ammoniumchloridlösung hydrolysiert. Die wässrige Mischung wird mit Äther extrahiert, die vereinigten Ätherextrakte werden getrocknet, und der Äther wird sodann entfernt, wobei ein gelblich-grünes Öl zurückbleibt, das destillativ gereinigt wird. Als Rückstand erhält man dabei ein gelbes Öl, das nach einigem Stehen zu den hellgelben festem 1-Benzoyl-4-(2-fluorbenzyl)-4-piperidinol erstarrt.

b) Eine gerührte Suspension aus 1,4 g 50%igen Natriumhydrid in 50 ml Dimethylformamid und 10 ml Benzol wird mit einer Lösung von 6,9 g 1-Benzyl-4-(2-fluor-benzyl)-4-piperidinol in 25 ml Dimethylformamid und 10 ml Benzol versetzt. Die Mischung wird dann bei 110—120°C 5 Stunden lang mit Hilfe eines Luftkühlers erhitzt, um das Benzol verdampfen zu lassen. Daraufhin läßt man das Gemisch auf Raumtemperatur abkühlen, gibt die abgekühlte Mischung auf Eis, verdünnt mit Wasser und extrahiert mit Äther. Die Ätherphase wird nacheinander mit Wasser und gesättigter wässriger Kochsalzlösung gewaschen und getrocknet. Man entfernt den Äther aus der getrockneten Lösung, wobei ein Öl zurückbleibt, das beim Stehenlassen zu hellgelben Feststoff erstarrt. Der Feststoff wird in Äther gelöst, und die Lösung mit ätherischem Chlorwasserstoff behandelt, wobei ein Salz in Form eines weißen Feststoffes anfällt, das in Isopropylalkohol zu weißen 2,3-Dihydro-1'-benzylspiro[benzofuran-2,4'-piperidin]-hydrochlorid-Kristallen vom Schmelzpunkt 246—247°C umkristallisiert wird.

Analyse:
Berechnet für $C_{19}H_{21}NO \cdot HCl$:
72,24% C; 7,03% H; 4,44% N
Gefunden: 71,95% C; 7,06% H; 4,34% N

Beispiel 2

a) Gemäß Beispiel 1) wird eine 2-Fluorbenzylchloridprobe in ihr Grignardreagenz überführt und dann mit einer N-Benzyl-4-piperidonprobe zu 1-Methyl-4-(2-fluorbenzyl)-4-piperidinol umgesetzt. Schmelzpunkt 93—95°C (nach 2-maligem Umkristallisieren).

b) Eine Lösung von 12,5 g 1-Methyl-4-(2-fluorbenzyl)-4-piperidinol in 50 ml Dimethylformamid und 20 ml Benzol werden bei 120°C zu einer gerührten Suspension von 2,3 g einer 57%igen öligen Suspension von Natriumhydrid in 110 ml Dimethylformamid und 20 ml Benzol gegeben. Man rührt das Reaktionsgemisch 84 Stunden bei 120°C und läßt es sodann allmählich auf Zimmertemperatur abkühlen, woraufhin man es in 800 ml Eiswasser gießt und mit Äther extrahiert. Die vereinigten Ätherextrakte werden getrocknet, bevor der Äther unter Zurückbleiben eines hellgelben Öls verdampft. Das Öl wird in Äther gelöst und mit ätherischem Chlorwasserstoff in sein Hydrochlorid überführt. Das Salz wird zweimal aus einem Äthanol-Äther-Gemisch zu 2,3-Dihydro-1'-methylspiro[benzofuran-2,4'-piperidin]-hydrochlorid vom Schmelzpunkt 248—250°C umkristallisiert.

Analyse:
Berechnet für $C_{13}H_{17}NO \cdot HCl$:
65,13% C; 7,57% H; 5,84% N; 14,79% Cl.
Gefunden: 64,92% C; 7,51% H; 5,85% N; 14,92% Cl.

Beispiel 3

a) Eine 2-Fluorbenzylchloridprobe wird in ihr Grignard-Reagenz überführt, welches nach der in Beispiel 1a) beschriebenen Methode mit 1-(β-Phenäthyl)-4-piperidon zu 1-(β-Phenäthyl)-4-(2-fluorbenzyl)-4-piperidinol umgesetzt wird. Man löst die freie Base in Äther und behandelt die Lösung mit ätherischem Chlorwasserstoff, wobei man nach zweimaligem Umkristallisieren aus Äthanol das Salz vom Schmelzpunkt 187—189°C erhält.

b) Eine Lösung von 34,3 g 1-(β-Phenäthyl)-4-(2-fluorbenzyl)-4-piperidinol in 250 ml Dimethylformamid wird zu einer gerührten Suspension von 3,2 g eines 99%igen Natriumhydrids in 250 ml Dimethylformamid gegeben. Das Reaktionsgemisch wird 3 Stunden bei 120°C gerührt, danach läßt man es auf Raumtemperatur abkühlen und gießt es in 750 ml Eiswasser. Das Zweiphasen-Gemisch wird dreimal mit Äther extrahiert, die vereinigten Ätherextrakte werden getrocknet und sodann zu einem hellgelben Öl konzentriert. Man löst das Öl in wenig Äther, wobei es in sein Hydrochlorid, einen weißen Niederschlag, überführt wird. Man sammelt das Salz und fällt es zweimal aus Äthanol zu 2,3-

Dihydro - 1' - ($\beta$ - phenäthyl)spiro[benzofuran - 2,4' - piperidin] - hydrochlorid vom Schmelzpunkt 270—272°C um.

Analyse:
Berechnet für $C_{20}H_{23}NO \cdot HCl$:
72,82% C; 7,33% H; 4,24% H; 10,74% Cl.
Gefunden: 72,65% C; 7,46% H; 4,18% H; 10,76% Cl.

Beispiel 4

a) Eine Suspension aus 3,4 g Magnesiumspänen in 50 ml Äther, wird mit einem Jodkristall und sodann mit ein paar Millilitern einer Lösung von 20,6 g 2-Fluorbenzylchlorid in 100 ml Äther versetzt. Daraufhin wird die Reaktion durch Erwärmen mit heißer Luft in Gang gesetzt, und während man das Gemisch auf Rückflußtemperatur hält, wird die restliche 2-Fluorbenzylchlorid-Äther-Lösung zugetropft. Nach beendeter Zugabe rührt man das Gemisch noch eine Stunde unter Rückfluß und fügt dann nacheinander 100 ml Äther sowie tropfenweise unter heftigem Rühren eine Lösung von 25,0 g N-Benzyl-3-pyrrolidon in 100 ml Äther zu. Nach beendeter Zugabe wird die so erhaltene Suspension weitere 2 Stunden am Rückfluß gekocht, weitere 16 Stunden bei Raumtemperatur gerührt und sodann gefiltert. Man wäscht den Filterkuchen gründlich mit Äther, woraufhin er durch Einrühren in eine Eis-Ammoniumchloridlösung hydrolysiert wird. Man extrahiert die wässrige Lösung dreimal mit Äther, wäscht die vereinigten Ätherextrakte nacheinander mit Wasser und einer Kochsalz-Lösung und trocknet das Produkt. Das Lösungsmittel wird entfernt und der Rückstand bei 135—140°C und einem Druck von 0,1 mmHg destilliert, worauf man ein zähes gelbes Öl erhält, welches beim Stehenlassen erstarrt. Der Feststoff wird zweimal aus einem Äthanol-Wasser-Gemisch zu 1-Benzyl-3-(2-fluorbenzyl)-3-pyrrolidinol vom Schmelzpunkt 75—77°C umkristallisiert.

b) 38,1 Gramm in 125 ml Benzol gelöstes 1-Benzyl-4-(2-fluorbenzyl)-3-pyrrolidinol und 125 ml Dimethylformamid werden bei 90°C zu einer gerührten Suspension von 4,7 g Natriumhydrid in 125 ml Benzol und 125 ml Dimethylformamid zugetropft. Nach beendeter Zugabe rührt man das Gemisch 120 Stunden bei 90°C und läßt es sodann auf Raumtemperatur abkühlen, bevor man es in 1 Liter Eiswasser gießt. Man extrahiert das Zweiphasen-Gemisch mit Äther und trocknet die vereinigten Ätherextrakte, die zu einem dunklen Öl eingedampft werden. Das Öl wird bei einer Temperatur von 170—175°C und einem Druck von 0,1 mmHg destilliert, wobei 24 g eines hellgelben Öls zurückbleiben, das nach einigem Stehen erstarrt. Der Feststoff wird zweimal aus Isopropylalkohol umkristallisiert, wobei man 2,3-Dihydro-1'-benzylspiro[benzofuran-2,3'-pyrrolidin] vom Schmelzpunkt 43—45°C als Produkt erhält.

Analyse:
Berechnet für $C_{18}H_{19}NO$:
81,47% C; 7,22% H; 5,28% N.
Gefunden: 81,59% C; 7,39% H; 5,16% N.

Beispiel 5

a) Man überführt eine 4-Chlor-2-fluorbenzylbromidprobe in ihr Grignard-Reagenz, das gemäß Beispiel 4a) mit N-Benzyl-4-piperidon zu 1-Benzyl-4-(4-chlor-2-fluorbenzyl)-4-piperidinol umgesetzt wird. Man destilliert das so erhaltene Produkt bei 175—180°C une einem Druck von 0,18 mmHg und erhält ein orangefarbenes Öl, welches in Äther in sein Hydrochlorid überführt wird. Das Salz wird viermal aus einer Äthylalkohol-Äther-Mischung umkristallisiert, wobei man das gereinigte Salz vom Schmelzpunkt 211,5—213°C erhält.

b) Eine Lösung aus 8,3 g 1-Benzyl-4-(4-chlor-2-fluorbenzyl)-4-piperidinol in 75 ml Benzol wird zu einer bei Raumtemperatur gehaltenen und gerührten Suspension aus 1,5 g Natriumhydrid (50%ige Öldispersion) in 100 ml Benzol zugetropft. Nach beendeter Zugabe erhitzt man das Reaktionsgemisch auf Rückflußtemperatur und fügt 35 ml Dimethylformamid hinzu. Danach wird das Gemisch nacheinander 15 Minuten am Rückfluß gekocht, auf Raumtemperatur abgekühlt und durch die tropfenweise Zugabe von 100 ml Wasser verdünnt. Das Reaktionsgemisch wird auf 1 Liter Eiswasser gegeben und dreimal mit Äther extrahiert. Man wäscht die vereinigten Ätherextrakte nacheinander mit einer gesättigten Kochsalzlösung, trocknet sie und entfernt den Äther unter vermindertem Druck, wobei ein Öl zurückbleibt. Das Öl wird in Hexan gekocht, das Hexan entfernt, und man erhält ein gelbes Öl, das nach Stehenlassen erstarrt. Man löst das Öl in Äther, wobei es in sein Hydrochlorid überführt wird, welches dreimal aus einer Äthylalkohol-Äther-Mischung umkristallisiert wird und somit das Produkt 2,3-Dihydro - 1' - benzyl - 6 - chlorspiro[benzofuran - 2,4' - piperidin] - hydrochlorid vom Schmelzpunkt 257—259°C ergibt.

Analyse:
Berechnet für $C_{19}H_{20}ClNO \cdot HCl$:
65,15% C; 6,04% H; 4,00% N; 20,24% Cl.
Gefunden: 65,01% C; 6,07% H; 4,05% N; 20,11% Cl.

Beispiel 6

a) Man überführt eine 5-Chlor-2-fluorbenzylbromidprobe in ihr Grignard-Reagenz, das gemäß Beispiel 4a) mit N-Benzyl-4-piperidon zu 1-Benzyl-4-(5-chlor-2-fluorbenzyl)-4-piperidinol umgesetzt wird. Man destilliert das Produkt bei 210°C und 0,15 mmHg, wobei ein orangefarbenes Öl zurückbleibt. Man löst das Öl in Äther, wobei es in sein Hydrochlorid überführt wird; es wird dann dreimal aus einem Äthylalkohol-Gemisch umkristallisiert und ergibt das Produkt 1-Benzyl-4-(5-chlor-2-fluorbenzyl)-4-piperidinol-hydrochlorid, Schmelzpunkt 217—219°C.

b) Man behandelt eine 1-Benzyl-4-(5-chlor-2-fluorbenzyl)-4-piperidinolprobe, die freie Base von a), wie in Beispiel 5b) beschrieben und erhält das Salz, 2,3-Dihydro-1'-benzyl-5-chlorspiro[benzofuran-2,4'-piperidin]-hydrochlorid.

Analyse:
Berechnet für $C_{19}H_{20}ClNO \cdot HCl$:
65,15% C; 6,04% H; 4,00% N; 20,24% Cl.
Gefunden: 65,21% C; 6,11% H; 3,98% N; 20,06% Cl.

Beispiel 7

Eine Lösung aus 5,3 g 2,3-Dihydro-1'-benzylspiro[benzofuran-2,4'-piperidin], der freien Base der Verbindung aus Beispiel 1, in 250 ml Isopropylalkohol wird in einem Schüttelapparat nach Paar unter 3,5 atü Wasserstoff bei 65—70°C in Gegenwart von 1 g eines 10%igen Palladium/Kohlenstoff-Katalysators bis zur vollständigen Wasserstoffaufnahme hydriert. Dann läßt man die Lösung auf Raumtemperatur abkühlen, filtriert und dampft sie zur Trockne ein, wobei ein weißer Feststoff zurückbleibt. Man löst den Feststoff in einem Benzol-Äther-Gemisch, filtriert die Lösung durch Celite und engt sie wieder ein, wobei man einen weißen Feststoff erhält, der nach Verreiben mit Äther das Produkt 2,3-Dihydrospiro[benzofuran-2,4'-piperidin], Schmelzpunkt 56—58,5°C, ergibt.

Analyse:
Berechnet für $C_{12}H_{15}NO$:
76,14% C; 8,00% H; 7,40% N.
Gefunden: 76,05% C; 8,08% H; 7,27% N.

Beispiel 8

Eine Lösung aus 21,4 g 2,3-Dihydro-1'-benzylspiro[benzofuran-2,3'-pyrrolidon], der freien Base der Verbindung aus Beispiel 4, in 250 ml Isopropylalkohol wird unter 3,2 atü Wasserstoff bei 50°C in Gegenwart von 2,0 g eines 10%igen Palladium/Kohlenstoff-Katalysators bis zur vollständigen Wasserstoffaufnahme hydriert. Dann filtriert man das Reaktionsgemisch und engt es unter vermindertem Druck ein, wobei ein gelbes Öl erhalten wird, das man mit Äther löst und somit in sein Hydrochlorid überführt. Das Salz wird zweimal aus einem Äthylalkohol-Äther-Gemisch umkristallisiert und ergibt das Produkt 2,3-Dihydrospiro[benzofuran-2,3'-pyrrolidin]-hydrochlorid vom Schmelzpunkt 174—178°C.

Analyse:
Berechnet für $C_{11}H_{13}NO \cdot HCl$:
62,23% C; 6,70% H; 6,65% N; 16,83% Cl.
Gefunden: 62,17% C; 6,72% H; 6,58% N; 16,60% Cl.

Beispiel 9

Eine gerührte Lösung aus 6,1 g 2,3-Dihydro-1'-benzyl-6-chlorspiro[benzofuran-2,4'-piperidin], der freien Base der Verbindung aus Beispiel 5, und 2,5 g Chlorameisensäure-äthylester in 150 ml Benzol wird 18 Stunden am Rückfluß gekocht. Dann läßt man die Lösung auf Raumtemperatur abkühlen, wäscht sie mit Wasser und einer gesättigten Natriumbicarbonatlösung und sodann mit gesättigter Kochsalzlösung, trocknet sie und dampft sie zur Trockne zu einem dunklen Öl, 2,3-Dihydro-6-chlor-1'-äthoxycarbonylspiro[benzofuran-2,4'-piperidin], ein. Das Öl wird in einem Gemisch aus 50 ml einer 50%igen Kalilauge und 100 ml Äthylalkohol aufgenommen, die erhaltene Mischung wird 18 Stunden am Rückfluß erhitzt und dann auf Raumtemperatur abgekühlt, bevor man dem Äthylalkohol unter vermindertem Druck entfernt. Die verbleibende wässrige Suspension wird mit Äther extrahiert, und die vereinigten Ätherextrakte werden mit 3n Salzsäure gewaschen. Die angesäuerte Reaktionslösung wird mit 6n Natronlauge basisch gestellt und mit Äther extrahiert. Man trocknet die vereinigten Ätherextrakte und engt sie sodann zur Trockne ein, wobei ein rohweißer Feststoff anfällt. Man löst den Feststoff in Äther, wobei er in sein Hydrochlorid überführt wird, welches man zweimal aus Äthylalkohol zu 2,3-Dihydro-6-chlorospiro[benzofuran-2,4'-piperidin]-hydrochlorid vom Schmelzpunkt 281—288°C umkristallisiert.

Analyse:
Berechnet für $C_{12}H_{14}ClNO \cdot HCl$:
55,40% C; 5,81% H; 5,38% N; 27,26% Cl.
Gefunden: 55,21% C; 5,86% H; 5,38% N; 27,07% Cl.

Beispiel 10

Man behandelt eine Probe von 2,3-Dihydro-1'-benzyl-5-chlorspiro[benzofuran-2,4'-piperidin], der freien Base der Verbindung aus Beispiel 6, gemäß dem Verfahren in Beispiel 9 und erhält das Produkt 2,3-Dihydro-5-chlorspiro[benzofuran-2,4'-piperidin]-hydrochlorid, Schmelzpunkt 217—218°C. Man erhält dieses Produkt nach dreimaligem Umkristallisieren, zweimal aus Äthylalkohol und danach einmal aus einem Äthylalkohol-Äther-Gemisch.

Analyse:
Berechnet für $C_{12}H_{14}ClNO \cdot HCl$:
55,40% C; 5,81% H; 5,38% N; 27,26% Cl.
Gefunden: 55,53% C; 5,84% H; 5,40% N; 27,11% Cl.

Beispiel 11

3,0 Gramm Allylbromid werden zu einem gerührten Gemisch aus 4,0 g 2,3-Dihydrospiro[benzofuran-2,4'-piperidin] aus Beispiel 7, das in 100 ml Isopropylalkohol gelöst wurde, und 13,8 g Kaliumcarbonat zugetropft. Nach beendeter Zugabe wird das Reaktionsgemisch 6 Stunden bei Rückflußtemperatur gehalten und dann 48 Stunden bei Raumtemperatur gerührt. Anschließend wird das Gemisch filtriert und das Filtrat zur Trockne eingedampft, wobei ein hellgelbes Öl zurückbleibt. Das Öl wird in Äther gelöst, wobei es in sein Hydrochlorid, einen weißen Niederschlag, überführt wird. Das Salz wird zweimal aus einem Äthylalkohol-Äther-Gemisch umkristallisiert, wobei 2,3-Dihydro-1'-allylspiro-[benzofuran-2,4'-piperidin]hydrochlorid vom Schmelzpunkt 216—217°C anfällt.

Analyse:
Berechnet für $C_{15}H_{19}NO \cdot HCl$
67,78% C; 7,59% H; 5,27% N; 13,47% Cl.
Gefunden: 67,72% C; 7,65% H; 5,18% N; 13,55% Cl.

Beispiel 12

1,8 Gramm Cyclopropylcarbonylchlorid werden zu einer gerührten Lösung von 0°C aus 3,2 g 2,3-Dihydro-spiro[benzofuran-2,4'-piperidin] in 50 ml Pyridin zugetropft. Nach beendeter Zugabe rührt man das Reaktionsgemisch 60 Stunden bei Raumtemperatur und erhitzt es 3 Stunden am Rückfluß. Anschließend läßt man das Gemisch auf Raumtemperatur abkühlen, gibt es auf 500 ml Wasser, stellt mit 20%iger Natronlauge alkalisch und entfernt das Lösungsmittel unter vermindertem Druck. Man verreibt den Rückstand mit 500 ml Wasser, extrahiert zweimal mit Essigsäureäthylester, trocknet die vereinigten Extrakte und entfernt das Lösungsmittel unter vermindertem Druck, wobei ein Feststoff anfällt. Dieser Feststoff wird dreimal aus Wasser umkristallisiert und ergibt 2,3-Dihydro-1'-cyclopropylcarbonylspiro[benzofuran-2,4'-piperidin] vom Schmelzpunkt 111—113°C in Form von weißen Flocken.

Analyse:
Berechnet für $C_{16}H_{19}NO_2$:
74,68% C; 7,44% H; 5,44% N.
Gefunden: 74,57% C; 7,47% H; 5,39% N.

Beispiel 13

Eine Lösung aus 3,6 g 4-Chlorphenylacetylchlorid in 10 ml Chloroform wird zu einer gerührten Suspension von 3,1 g 2,3-Dihydrospiro[benzofuran-2,4'-piperidin] aus Beispiel 7 und 7 g Natriumbicarbonat in 40 ml Chloroform zugetropft. Nach beendeter Zugabe wird das Reaktionsgemisch 20 Stunden bei Raumtemperatur gerührt und dann 24 Stunden am Rückfluß erhitzt. Anschließend kühlt man das Gemisch auf Raumtemperatur ab, filtriert es, wäscht es nacheinander mit 3n Salzsäure, einer gesättigten Natriumbicarbonat-Lösung und einer gesättigten Kochsalzlösung und trocknet es, woraufhin es zu einem dunkeln Öl eingedampft wird. Man verreibt das Öl viermal mit 75 ml kochendem Hexan. Die kombinierten Verreibungsprodukte werden gesammelt und abgekühlt, wobei ein weißer Niederschlag zurückbleibt, der durch Filtration gesammelt wird. Der Niederschlag wird dreimal aus Hexan umkristallisiert und ergibt das Produkt 2,3-Dihydro-1'-(4-chlorphenylacetyl)spiro[benzofuran-2,4'-piperidin] vom Schmelzpunkt 108—110°C.

Analyse:
Berechnet für $C_{20}H_{20}ClNO_2$:
70,27% C; 5,90% H; 4,10% N; 10,37% Cl.
Gefunden: 70,10% C; 5,94% H; 4,23% N; 10,22% Cl.

Beispiel 14

Eine gerührte Suspension von 3,5 g 2,3-Dihydrospiro[benzofuran-2,4'-piperidin] aus Beispiel 7 und 7,5 g Natriumbicarbonat in 40 ml Chloroform wird mit einer Lösung aus 4,4 g 4-Methoxyphenylacetylchlorid in 10 ml Chloroform umgesetzt und anschließend nach dem in Beispiel 13 beschriebenen Verfahren behandelt, so daß ein gelbes Öl entsteht. Dieses Öl wird an einer Silicagekolonne mit einem 3%igen Methylalkohol in Chloroform als Eluiermittel chromatographiert, wobei man einen weißen Feststoff erhält, der dreimal aus Hexan umkristallisiert wird und das Produkt 2,3-Dihydro-1'-[(4-methoxyphenyl)acetyl]-spiro[benzofuran-2,4'-piperidin] vom Schmelzpunkt 87—88°C ergibt.

Analyse:
Berechnet für $C_{21}H_{23}NO_3$:
74,75% C; 6,87% H; 4,15% N.
Gefunden: 74,62% C; 6,96% H; 4,11% N.

Beispiel 15

4,0 Gramm 1-Brom-3-methyl-2-buten in 20 ml Dimethylformamid werden zu einer gerührten Suspension von 5,0 g 2,3-Dihydrospiro[benzofuran-2,4'-piperidin], 10 g Kaliumcarbonat und einigen Gramm Kaliumjodid in 100 ml wasserfreiem Dimethylformamid zugetropft. Nach beendeter Zugabe wird das Reaktionsgemisch nacheinander 2 Stunden am Rückfluß erhitzt, 16 Stunden bei Raumtemperatur gerührt, filtriert und in Wasser gegossen. Man extrahiert das Zweiphasengemisch dreimal mit je 150 ml Äther, danach werden die vereinigten Ätherextrakte getrocknet und mit ätherischer Salzsäure behandelt, wobei ein Niederschlag entsteht. Der Niederschlag wird gesammelt und zweimal aus einem Äthylalkohol-Äther-Gemisch und einmal aus Äthylalkohol umkristallisiert, wobei man das Produkt 2,3-Dihydro-1'-(3-methyl-2-butenyl)-spiro[benzofuran-2,4'-piperidin]-hydrochlorid vom Schmelzpunkt 227—229°C erhält.

Analyse:
Berechnet für $C_{17}H_{23}NO \cdot HCl$:
60,49% C; 8,23% H; 4,76% N.
Gefunden: 69,57% C; 8,25% H; 4,76% N.

Beispiel 16

3 Milliliter Acetylchlorid in 10 ml Chloroform werden zu einer gerührten Suspension von 6,0 g 2,3-Dihydrospiro[benzofuran-2,4'-piperidin] aus Beispiel 7 und 14,0 g Natriumbicarbonat in 75 ml Chloroform zugetropft. Nach beendeter Zugabe wird das Reaktionsgemisch 16 Stunden bei Raumtemperatur gerührt, filtriert, nacheinander mit Wasser, verdünnter Salzsäure und einer gesättigten Kochsalzlösung gewaschen, getrocknet, und das Lösungsmittel unter vermindertem Druck entfernt, wobei ein Rückstand erhalten wird, der nach Stehenlassen erstarrt. Dieser Feststoff wird zweimal aus Hexan zum Produkt 2,3-Dihydro-1'-acetylspiro[benzofuran-2,4'-piperidin] vom Schmelzpunkt 94—97°C umkristallisiert.

Analyse:
Berechnet für $C_{14}H_{17}NO_2$:
72,70% C; 7,41% H; 6,05% N.
Gefunden: 72,85% C; 7,47% H; 6,08% N.

Beispiel 17

Eine Mischung aus 4,2 g Cyclobutylcarbonylchlorid in 10 ml Chloroform wird zu einem gerührten Gemisch aus 6,0 g 2,3-Dihydrospiro[benzofuran-2,4'-piperidin] aus Beispiel 7 und 20,0 g Kaliumcarbonat in 75 ml Chloroform zugetropft. Nach beendeter Zugabe wird das Reaktionsgemisch nacheinander 24 Stunden am Rückfluß erhitzt, gekühlt, filtriert und in Wasser eingetragen. Man wäscht das Zweiphasengemisch zuerst mit 3n Salzsäure, dann mit 3n Natronlauge und schließlich mit einer gesättigten Kochsalzlösung, trocknet es, entfernt das Lösungsmittel und erhält dann einen festen Rückstand. Der Rückstand wird zweimal aus einem Benzol-Hexan-Gemisch umkristallisiert, wobei das Produkt 2,3-Dihydro-1'-cyclobutylcarbonylspiro[benzofuran-2,4'-piperidin] vom Schmelzpunkt 91—93°C entsteht.

Analyse:
Berechnet für $C_{17}H_{21}NO_2$:
75,25% C; 7,80% H; 5,16% N.
Gefunden: 75,15% C; 7,88% H; 5,14% N.

Beispiel 18

Eine Mischung aus 4,7 g Äthyloxalylchlorid in 15 ml Chloroform wird zu einer gerührten Mischung aus 5,0 g 2,3-Dihydrospiro[benzofuran-2,4'-piperidin] aus Beispiel 7 und 10,0 g Natriumbicarbonat in 50 ml Chloroform zugetropft. Nach beendeter Zugabe wird das Reaktionsgemisch nacheinander 3 Stunden am Rückfluß erhitzt, 16 Stunden bei Raumtemperatur gerührt, filtriert, zuerst mit Wasser, dann mit 3n Salzsäure und schließlich mit einer gesättigten Natriumbicarbonatlösung sowie mit einer gesättigten Kochsalzlösung gewaschen, getrocknet und das Lösungsmittel unter vermindertem Druck entfernt, wobei ein gelbbrauner Feststoff anfällt. Dieser Feststoff wird an einer Silicagelkolonne mit 2% Methylalkohol in Chloroform als Eluiermittel chromatographiert, um das Produkt zu reinigen, welches aus Hexan zu 2,3-Dihydro-1'-äthoxyoxalylspiro[benzofuran-2,4'-piperidin] vom Schmelzpunkt 87—91°C mit der Formel

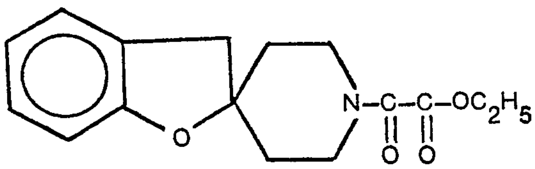

umkristallisiert wird.

Analyse:
Berechnet für $C_{16}H_{19}NO_4$:
66,42% C; 6,62% H; 4,84% N.
Gefunden: 66,51% C; 6,60% H; 4,84% N.

Beispiel 19

Eine Probe von 5,8 g 2,3-Dihydrospiro[benzofuran-2,4'-piperidin] aus Beispiel 7 wird nach Beispiel 18 mit 4,8 g 2-Furoylchlorid behandelt, wobei ein rohweißer Feststoff entsteht. Dieser Feststoff wird durch dreimaliges Umkristallisieren aus Hexan gereinigt und ergibt das Produkt 2,3-Dihydro-1'-(2-furoyl)-spiro[benzofuran-2,4'-piperidin] vom Schmelzpunkt 102—103°C.

Analyse:
Berechnet für $C_{17}H_{17}NO_3$:
72,07% C; 6,05% H; 4,94% N.
Gefunden: 72,22% C; 6,10% H; 4,91% N.

Beispiel 20

6,0 Gramm 3-Phenoxypropylbromid in 30 ml Dimethylformamid werden zu einer gerührten Mischung aus 5,0 g 2,3-Dihydrospiro[benzofuran-2,4'-piperidin] aus Beispiel 7, 10 g Kaliumcarbonat und 1 g Kaliumjodid zugetropft. Nach beendeter Zugabe wird das Reaktionsgemisch 3 Stunden am Rückfluß erhitzt, auf Raumtemperatur abgekühlt, filtriert und in Wasser eingetragen. Man extrahiert das Zweiphasengemisch mit Äther und wäscht die vereinigten Ätherextrakte mit einer gesättigten Kochsalzlösung, dann trocknet man sie und entfernt das Lösungsmittel, wobei ein gelbbraunes Öl zurückbleibt. Man löst das Öl in Äther, wobei es in sein Hydrochlorid überführt wird. Dieses wird gesammelt und zweimal aus Äthylalkohol umkristallisiert, woraufhin das Produkt 2,3-Dihydro-1'-(3-phenoxypropyl)spiro[benzofuran-2,4'-piperidin]-hydrochlorid vom Schmelzpunkt 220—223°C erhalten wird.

Analyse:
Berechnet für $C_{21}H_{25}NO_2 \cdot HCl$:
70,08% C; 7,28% H; 3,89% N.
Gefunden: 70,40% C; 7,34% H; 3,82% N.

Beispiel 21

Man behandelt eine gerührte Mischung aus 5,0 g 2,3-Dihydrospiro[benzofuran-2,4'-piperidin] aus Beispiel 7 und 15 g Natriumbicarbonat in 75 ml Chloroform mit einer Mischung aus 8,8 g 3,4,5-Trimethoxybenzoylchlorid in 125 ml Chloroform gemäß dem in Beispiel 7 beschriebenen Verfahren und erhält so einen gelbbraunen Feststoff. Dieser Feststoff wird nacheinander aus Äthylalkohol umkristallisiert, an einer Silicagelkolonne mit 2% Methylalkohol in Chloroform als Eluiermittel chromatographiert und aus Äthylalkohol umkristallisiert, so daß sich das Produkt 2,3-Dihydro-1'-(3,4,5-trimethoxybenzoyl)spiro[benzofuran-2,4'-piperidin] vom Schmelzpunkt 148—150°C ergibt.

Analyse:
Berechnet für $C_{22}H_{25}NO_5$:
68,91% C; 6,57% H; 3,65% N.
Gefunden: 69,01% C; 6,61% H; 3,54% N.

Beispiel 22

6,1 Gramm 1-(Indol-3-glyoxylyl)chlorid) werden vorsichtig portionsweise zu einer gerührten Mischung aus 4,0 g 2,3-Dihydrospiro[benzofuran-2,4′-piperidin] aus Beispiel 7 in 75 ml Chloroform und 5,0 g Kaliumcarbonat in 50 ml Wasser gegeben. Nach beendeter Zugabe rührt man das Reaktionsgemisch 24 Stunden bei Raumtemperatur und trennt dann die organische von der wässrigen Phase. Man extrahiert die wässrige Phase mit Chloroform und wäscht die vereingten Extrakte nacheinander mit Wasser, 3n Salzsäure und einer gesättigten Natriumbicarbonatlösung, dann trocknet man das Gemisch und entfernt das Lösungsmittel, wobei ein Feststoff zurückbleibt. Dieser Feststoff wird an einer Silicagelkolonne mit 2% Methylalkohol in Chloroform als Eluiermittel chromatographiert und dann aus einem Äthylalkohol-Wasser-Gemisch zu 2,3-Dihydro-1′-(indol-3-ylglyoxylolyl)spiro[benzofuran-2,4′-piperidin] vom Schmelzpunkt 226—228°C mit der Formel

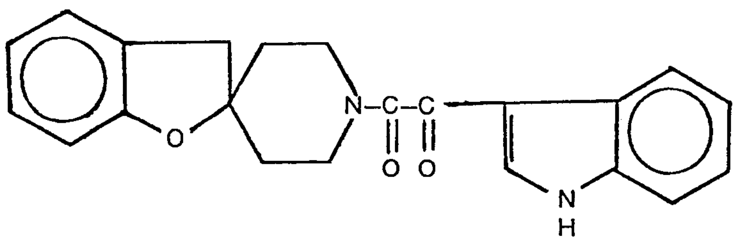

umkristallisiert.

Analyse:
Berechnet für $C_{22}H_{20}N_2O_3$:
73,32% C; 5,59% H; 7,77% N.
Gefunden: 73,31% C; 5,67% H; 7,87% N.

Beispiel 23

Eine Lösung aus 4,0 g Phenylacetylchlorid in 10 ml Chloroform wird zu einer gerührten Lösung von 4,0 g 2,3-Dihydro-spiro[benzofuran-2,3′-pyrrolidin], der freien Base der Verbindung aus Beispiel 8, und 3 ml Triäthylamin in 40 ml Chloroform zugetropft. Nach beendeter Zugabe rührt man das Reaktionsgemisch 18 Stunden bei Raumtemperatur und 3 Stunden am Rückfluß und läßt es sodann auf Raumtemperatur abkühlen, bevor man es in 100 ml Wasser einträgt. Man sammelt die organische Phase und wäscht sie nacheinander mit verdünnter Säure, verdünnter Base und einer gesättigten Kochsalzlösung und trocknet sie anschließend. Danach wird das Lösungsmittel abgedampft, wobei ein dunkles Öl zurückbleibt, das man an einer Silicagelkolonne mit 5% Methylalkohol in Chloroform als Eluiermittel chromatographiert, woraufhin ein hellgelbes Öl entsteht. Man verreibt dieses Öl mit kochendem Hexan, dekantiert die Flüssigkeit und läßt sie abkühlen, wobei ein Niederschlag erhalten wird. Man sammelt den Niederschlag und kristallisiert ihn zweimal aus Hexan um, um so das Reinprodukt, 2,3-Dihydro-1′(phenylacetyl)spiro[benzofuran-2,3′-pyrrolidin], vom Schmelzpunkt 98—99°C zu erhalten.

Analyse:
Berechnet für $C_{19}H_{19}NO_2$:
77,79% C; 6,53% H; 4,77% N.
Gefunden: 77,55% C; 6,72% H; 4,72% N.

Beispiel 24

Eine Lösung aus 3,2 g 2,3-Dihydro-1′-cyclopropylcarbonylspiro[benzofuran-2,3′-piperidin] aus Beispiel 12 in 75 ml Tetrahydrofuran wird zu einer gerührten Suspension von 0,60 g Lithium-Aluminium-Hydrid in 50 ml Tetrahydrofuran zugetropft. Nach beendeter Zugabe wird das Reaktionsgemisch 4 Stunden am Rückfluß enhitzt und dann 16 Stunden bei Raumtemperatur gerührt. Das überschüssige Lithium-Aluminium-Hydrid wird durch die tropfenweise Zugabe von 75 ml Wasser zerstört, bevor man das Reaktionsgemisch filtriert. Das Filtrate wird zuerst mit Wasser gewaschen und dann mit Chloroform extrahiert. Die vereinigten Chloroformextrakte werden getrocknet und zur Trockne eingedampft, wobei ein gelbes Öl zurückbleibt, das man in Äther löst, wobei es in sein Hydrochlorid überführt wird. Dieses Salz wird zweimal aus einem Äthylalkohol-Äthergemisch umkristallisiert, so daß das Produkt 2,3-Dihydro - 1′ - cyclopropylmethylspiro[benzofuran - 2,4′ - piperidin]hydrochlorid vom Schmelzpunkt 221—223°C entsteht.

Analyse:
Berechnet für $C_{16}H_{21}NO \cdot HCl$:
68,68% C; 7,93% H; 5,01% N; 12,67% Cl.
Gefunden: 68,52% C; 8,03% H; 4,95% N; 12,60% Cl.

Beispiel 25

Eine Lösung aus 10 ml VITRIDE in 10 ml Benzol wird zu einer gerührten Lösung aus 4,7 g 2,3-Dihydro-1′-[(4-methoxyphenyl)acetyl]spiro[benzofuran-2,4′-piperidin]aus Beispiel 14 in 40 ml Benzol zugetropft. Nach beendeter Zugabe erhitzt man das Reaktionsgemisch am Rückfluß, bis die Reduktion abgeschlossen ist. Danach kühlt man die Mischung in einem Eisbad und fügt tropfenweise 15 ml Wasser und dann 15 ml 6n Natronlauge hinzu. Man extrahiert die wässrige Phase mit 150 ml Benzol, vereinigt den Extrakt mit der organischen Phase, wäscht die gesamte organische Lösung mit einer gesättigten Kochsalzlösung und trocknet sie. Das Lösungsmittel wird abgedampft, wobei ein hellgelbes Öl zurückbleibt, das man in Äther löst, wobei es in sein Hydrochlorid überführt wird. Man sammelt das Salz durch Filtrieren und kristallisiert es zweimal aus Äthylakohol und danach einmal aus einem Äthylalkohol-Äther-Gemisch um, wobei das Produkt 2,3-Dihydro-1′-(4-methoxy-β-phenäthyl)-spiro[benzofuran-2,4′-piperidin]-hydrochlorid von Schmelzpunkt 274—275°C entsteht.

Analyse:
Berechnet für $C_{21}H_{25}NO_2 \cdot HCl$:
70,08% C; 7,28% H; 3,89% N; 9,85% Cl.
Gefunden: 69,95% C; 7,41% H; 3,96% N; 9,73% Cl.

Beispiel 26

Eine Lösung aus 3,7 g 2,3-Dihydro-1′-(phenylacetyl)spiro[benzofuran-2,3′-pyrrolidin] aud Beispiel 23 in 30 ml Tetrahydrofuran wird zu einer gerührten Suspension von 0,7 g Lithium-Aluminium-Hydrid in 40 ml Tetrahydrofuran zugetropft. Nach beendeter Zugabe wird das Reaktionsgemisch 6 Stunden am Rückfluß erhitzt und dann 18 Stunden bei Raumtemperatur gerührt, während man 50 ml Wasser zutropft, wobei das Reaktionsgemisch in einem Bad aus Eis und Methylalkohol gekühlt wird. Man filtriert das Reaktionsgemisch und dampft das Filtrat zur Trockne ein, wobei ein gelbes Öl zurückbleibt. Das Öl wird an einer Aluminiumoxidkolonne mit Äther als Eluiermittel chromatographiert, wobei man ein Öl erhält, das in Äther gelöst wird, woraufhin man es in sein Oxalat überführt. Das Salz wird gesammelt und dreimal aus Äthylalkohol umkristallisiert, wobei das Produkt 2,3-Dihydro-1′-(β-phenäthyl)-spiro[benzofuran-2,3′-pyrrolidin]-oxalat vom Schmelzpunkt 158—161°C anfällt.

Analyse:
Berechnet für $C_{19}H_{21}NO \cdot (CO_2H)_2$:
68,28% C; 6,28% H; 3,79% N.
Gefunden: 68,10% C; 6,43% H; 3,75% N.

Beispiel 27

Eine Lösung aus 3,9 g 2,3-Dihydro-1′-acetylspiro[benzofuran-2,4′-piperidin] aus Beispiel 16 in 50 ml Tetrahydrofuran wird zu einer gerührten Suspension von 0,7 g Lithium-Aluminium-Hydrid in 50 ml Tetrahydrofuran zugetropft. Nach beendeter Zugabe wird das Reaktionsgemisch zuerst 3 Stunden am Rückfluß erhitzt, dann 16 Stunden bei Raumtemperatur gerührt und in einem Eis-Salz-Bad unter der tropfenweise Zugabe von 30 ml Wasser gekühlt. Man filtriert das Reaktionsgemisch und wäscht den Filterkuchen mit Äther. Man trocknet die Ätherlösungen und fügt ätherischen Chlorwasserstoff hinzu, woraufhin ein Niederschlag gebildet wird. Man sammelt den Niederschlag durch Filtrieren der Lösung und kristallisiert ihn zweimal aus einem Äthylalkohol-Äther-Gemisch um, wobei 2,3-Dihydro-1′-äthylspiro[benzofuran-2,4′-piperidin]-hydrochlorid vom Schmelzpunkt 240—242°C erhalten wird.

Analyse:
Berechnet für $C_{14}H_{19}NO \cdot HCl$:
66,26% C; 7,94% H; 5,52% N.
Gefunden: 66,24% C; 7,97% H; 5,51% N.

Beispiel 28

Eine Lösung aus 7,3 g 2,3-Dihydro-1′(4-chlorophenylacetyl)spiro[benzofuran-2,4′-piperidin] aus Beispiel 13 in 100 ml Tetrahydrofuran wird unter einer Stickstoffatmosphäre zu 40 ml einer gerührten Lösung von Diboran (1,08 molar) in Tetrahydrofuran eingetropft. Nach beendeter Zugabe erhitzt man das Reaktionsgemisch zuerst 2 Stunden am Rückfluß, rührt dann 16 Stunden bei Raumtemperatur und tropft 30 ml 6n Salzsäure hinzu. Anschließend entfernt man das Lösungsmittel unter vermindertem Druck und stellt die so erhaltene Suspension mit 2n Natronlauge basisch. Man extrahiert die alkalisierte Mischung mit Chloroform, trocknet die vereinigten Chloroformextrakte und engt sie ein. Der Rückstand wird mit Äther verrieben, wobei ein Niederschlag entsteht, der

zweimal aus einem Äthylalkohol-Wasser-Gemisch zu weißen Nadeln aus 2,3-Dihydro-1'-(4-chlor-β-phenäthyl)spiro[benzofuran-2,4'-piperidin] vom Schmelzpunkt 64—66°C umkristallisiert wird.

Analyse:
Berechnet für $C_{20}H_{22}ClNO$:
73,27% C; 6,77% H; 4,27% N.
Gefunden: 73,16% C; 6,71% H; 4,22% N.

Beispiel 29

Eine Lösung aus 2,3-Dihydro-1'-cyclobutylcarbonylspiro[benzofuran-2,4'-piperidin] aus Beispiel 17 in 75 ml Tetrahydrofuran wird zu einer gerührten Suspension von 1,3 g Lithium-Aluminium-Hydrid in 75 ml Tetrahydrofuran zugetropft. Nach beendeter Zugabe wird das Reaktionsgemisch zuerst 2 Stunden am Rückfluß erhitzt, dann in einem Eisbad gekühlt und 20 ml Wasser zugetropft. Mann filtriert das Gemisch und wäscht den Filterkuchen gründlich mit Äther. Das ätherhaltige Waschwasser wird mit dem Filtrat vereinigt, welches dann unter vermindertem Druck eingeengt wird, wobei ein schwachgelbes Öl zurückbleibt. Man löst das Öl in Äther, wobei es in sein Hydrochlorid überführt wird. Das Salz wird gesammelt und zweimal aus einem Gemisch aus Äthylalkohol und Äther umkristallisiert, woraufhin das Produkt 2,3-Dihydro-1'-cyclobutylmethylspiro[benzofuran-2,4'-piperidin]-hydrochlorid vom Schmelzpunkt 226—228°C ensteht.

Analyse:
Berechnet für $C_{17}H_{23}NO \cdot HCl$:
69,49% C; 8,23% H; 4,67% N.
Gefunden: 69,25% C; 8,12% H; 4,82% N.

Beispiel 30

Eine Lösung aus 5,0 g 2,3-Dihydro-1'-äthyloxalylspiro[benzofuran-2,4'-piperidin] aus Beispiel 18 in 75 ml Tetrahydrofuran wird unter Stickstoffatmosphäre zu einer gerührten Suspension von 2,2 g Lithium-Aluminium-Hydrid in 50 ml Tetrahydrofuran eingetropft. Nach beendeter Zugabe erhitzt man das Reaktionsgemisch zuerst 3 Stunden am Rückfluß, rührt 16 Stunden bei Raumtemperatur, kühlt das Gemisch in einem Eisbad und tropft 20 ml Wasser hinzu. Man filtert das Gemisch, wäscht den Filterkuchen gründlich mit Äther und trocknet das Filtrat. Das Lösungsmittel wird unter vermindertem Druck entfernt, wobei ein gelber Feststoff zurückbleibt. Diesen löst man in Äther, wobei er in sein Hydrochlorid überführt wird. Das Salz wird aus einem Äthylalkohol-Äther-Gemisch zu 2,3-Dihydro-1'-(β-hydroxyäthyl)spiro[benzofuran-2,4'-piperidine]-hydrochlorid vom Schmelzpunkt 176—178°C umkristallisiert.

Analyse:
Berechnet für $C_{14}H_{19}NO_2 \cdot HCl$:
62,33% C; 7,47% H; 5,19% N.
Gefunden: 62,24% C; 7,56% H; 5,11% N.

Beispiel 31

Eine Lösung aus 6,5 g 2,3-Dihydro-1'-(2-furoyl)-spiro[benzofuran-2,4'-piperidin] aus Beispiel 19 in 75 ml Tetrahydrofuran wird zu einer gerührten Suspension von 1,1 g Lithium-Aluminium-Hydrid in 75 ml Tetrahydrofuran zugetropft. Nach beendeter Zugabe wird das Reaktionsgemisch 3 Stunden am Rückfluß erhitzt, 16 Stunden bei Raumtemperatur gerührt und in einem Eisbad gekühlt, wobei 20 ml Wasser zugetropft werden. Anschließend filtriert man das Gemisch, wäscht den Filterkuchen mit Äther, trocknet das Filtrat und dampft es unter vermindertem Druck zu einem gelben Öl ein, welches dann an einer Aluminiumoxidkolonne mit Ather als Eluiermittel zu einem milchigweißen Ol chromatographiert wird. Man löst dieses Öl in Äther, wobei es in sein Oxalat überführt wird. Das Salz wird gesammelt und zweimal aus Äthylakohol umkristallisiert, wodurch man das Produkt 2,3-Dihydro-1'-furfurylspiro[benzofuran-2,4'-piperidin]-oxalat vom Schmelzpunkt 145—147°C erhält.

Analyse:
Berechnet für $C_{17}H_{19}NO_2 \cdot (CO_2H_2)$:
63.50% C; 5,89% H; 3,90% N.
Gefunden: 63,73% C; 5,89% H; 3,82% N.

Beispiel 32

Gemäß dem Verfahren aus Beispiel 22 behandelt man eine gerührte Suspension von 1,5 g Lithium-Aluminium-Hydrid in 150 ml Tetrahydrofuran unter einer Stickstoffatomsphäre mit einer Lösung aus 2,3-Dihydro-1'-(indol-3-ylglyoxyloyl)spiro[benzofuran-2,4'-piperidin] aus Beispiel 22 in 75 ml Tetrahydrofuran und erhält so einen gelbbraunen Feststoff. Dieser Feststoff wird nacheinander aus einem Äthylalkohol-Wasser-Gemisch umkristallisiert, an einer Silicagelkolonne mit 2% Methylalkohol in Chloroform als Eluiermittel chromatographiert und aus Äthylalkohol zu 2,3-Dihydro-1'-[2-(3-indolyl)-äthyl]spiro[benzofuran-2,4'-piperidin] vom Schmelzpunkt 167—169°C umkristallisiert.

Analyse:
Berechnet für $C_{22}H_{24}N_2O$:
79,48% C; 7,28% H; 8,43% N.
Gefunden: 79,38% C; 7,34% H; 8,45% N.

Beispiel 33

Gemäß dem Verfahren aus Beispiel 27 behandelt man eine gerührte Suspension von 1,0 g Lithium-Aluminium-Hydrid in 150 ml Tetrahydrofuran unter einer Stickstoffatmosphäre mit einer Lösung aus 6,0 g 2,3-Dihydro-1'-(3,4,5-trimethoxybenzoyl)spiro[benzofuran-2,4'-piperidin] aus Beispiel 21 in 50 ml Tetrahydrofuran und erhält auf diese Weise ein hellgelbes Öl. Man löst dieses Öl in Äther, wobei es in sein Hydrochlorid überführt wird. Das Salz wird gesammelt und zweimal aus einem Äthylalkohol-Äther-Gemisch zum Produkt 2,3-Dihydro-1'-(3,4,5-trimethoxybenzyl)spiro[benzofuran-2,4'-piperidin]-hydrochlorid. Schmelzpunkt 218—219°C umkristallisiert.

Analyse:
Berechnet für $C_{22}H_{27}NO_4 \cdot HCl$:
65,10% C; 6,95% H; 3,45% N.
Gefunden: 64,92% C; 7,01% H; 3,42% N.

Beispiel 34

3,8 Gramm Salpetersäure (Dichte 1,42) in 30 ml Eisessig werden zu einer gerührten Lösung von 4,7 g 2,3-Dihydro-1'-acetylspiro[benzofuran-2,4'-piperidin] aus Beispiel 16 in 65 ml Eisessig zugetropft. Nach beendeter Zugabe erhitzt man das Reaktionsgemisch langsam 2 Stunden lang auf 100°C, dann kühlt man ab und gibt es auf 500 ml Wasser. Man extrahiert die verdünnte Mischung mit Chloroform und wäscht die Chloroformextrakte nacheinander mit einer gesättigten Natriumbicarbonatlösung und einer gesättigten Kochsalzlösung und trocknet das Produkt. Das Chloroform wird unter vermindertem Druck entfernt. Man verreibt den Rückstand mit Äther, wobei man einen orangefarbenen Niederschlag erhält, der gesammelt und dann zuerst aus einem Athylalkohol-Ather-Gemisch und anschließend aus Äthylalkohol umkristallisiert wird, woraufhin das Produkt 2,3-Dihydro-1'-acetyl-5-nitrospiro[benzofuran-2,4'-piperidin] von Schmelzpunkt 149—150°C anfällt.

Analyse:
Berechnet für $C_{14}H_{16}N_2O_4$:
60,86% C; 5,84% H; 10,14% N.
Gefunden: 60,83% C; 5,82% H; 10,14% N.

Beispiel 35

Eine Lösung aus 3,4 g 2,3-Dihydro-1'-acetyl-5-nitrospiro[benzofuran-2,4'-piperidin] in 150 ml 6n Salzsäure wird 45 Stunden am Rückfluß erhitzt und dann 16 Stunden bei Raumtemperatur gerührt. Anschließend extrahiert man das Reaktionsgemisch einmal mit Äther, stellt es mit 6n Natronlauge alkalisch und extrahiert es dreimal mit Äther. Man trocknet die vereinigten Ätherextrakte und entfernt das Lösungsmittel, wobei ein gelber Rückstand verbleibt. Dieser Rückstand wird in Äther gelöst, wobei er in sein Hydrochlorid überführt wird, Man samellt das Salz, trocknet es und kristallisiert es anschließend zweimal aus Äthylalkohol zu 2,3-Dihydro-5-nitrospiro[benzofuran-2,4'-piperidin]-hydrochlorid vom Schmelzpunkt 265—266°C um.

Analyse:
Berechnet für $C_{12}H_{14}N_2O_3 \cdot HCl$:
53,24% C; 5,59% H; 10,35% N.
Gefunden: 53,36% C; 5,77% H; 10,28% N.

**Patentansprüche**

1. Verbindungen der Formel

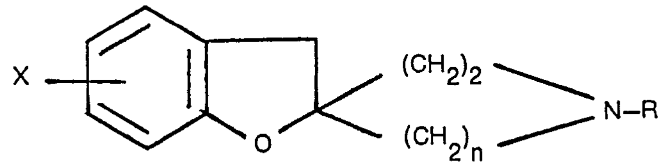

worin
X Wasserstoff, Nitro, Amino, Chlor, Fluor, Brom oder Methoxy,

R Wasserstoff, $C_{1-6}$-Alkyl, $C_{2-6}$-Alkenyl, Hydroxyäthyl, $C_{1-6}$-Alkanoyl, $C_{1-6}$-Alkoxyoxalyl, unsubstituiertes oder substituiertes Phenyl-$C_{1-6}$-alkanoyl, Benzoyl, Phenyl-$C_{1-6}$-alkyl oder Phenoxy-$C_{1-6}$-alkyl, wobei der Phenylrest jeweils ein oder zweifach mit Nitro, Amino-, Chlor, Fluor, Brom oder Methoxy substituiert sein kann, $C_{3-7}$-Cycloalkyl-$C_{1-6}$-alkyl, $C_{3-7}$-Cycloalkylcarbonyl, $C_{1-6}$-Alkoxycarbonyl, Phenoxycarbonyl, Furfuryl Furoyl, N-[2-(3-indolyläthyl] oder N-(indol-3-yl-oxalyl),

n 1 oder 2

bedeuten,

sowie deren pharmazeutisch unbedenkliches Säureadditionssalze.

2. 2,3-Dihydro-1′-methylspiro[benzofuran-2 4′-piperidin] oder dessen pharmazeutisch unbedenkliches Säureadditionssalz.

3. 2,3-Dihydro-1′-cyclobutylmethylspiro[benzofuran-2,4′-piperidin] oder dessen pharmazeutisch unbedenkliches Säureadditionssalz.

4. 2,3-Dihydro-1′-(β-phenyläthyl)spiro[benzofuran-2,4′-piperidin] oder dessen pharmazeutisch unbedenkliches Säureadditionssalz.

5. 2,3-Dihydro-1′ - (β - phenäthyl)spiro[benzofuran-2,3′-pyrrolidin] oder dessen pharmazeutisch unbedenkliches Säureadditionssalz.

6. 2,3-Dihydro-5-nitrospiro[benzofuran-2,4′-piperidin] oder dessen pharmazeutisch unbedenkliches Säureadditionssalz.

7. Verbindungen der Formel I zur Verwendung als Arzneimittel.

8. Verfahren zur Herstellung von Verbindungen der Formel I.

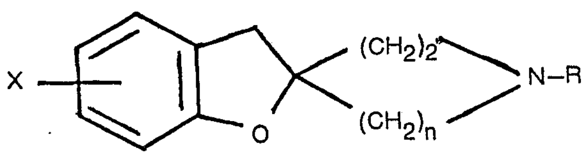

I

worin

X Wasserstoff, Nitro, Amino, Chlor, Fluor, Brom oder Methoxy,

R Wasserstoff, $C_{1-6}$-Alkyl, $C_{2-6}$-Alkenyl, Hydroxyäthyl, $C_{1-6}$-Alkanoyl, $C_{1-6}$-Alkoxyoxalyl, unsubstituiertes oder substituiertes Phenoxy-$C_{1-6}$-alkanoyl, Benzoyl, Phenyl-$C_{1-6}$-alkyl oder Phenoxy-$C_{1-6}$ alkyl, wobei der Phenylrest jeweils ein oder zweifach mit Nitro, Amino-, Chlor, Fluor, Brom oder Methoxy substituiert sein kann, $C_{3-7}$-Cycloalkyl-$C_{1-6}$-alkyl, $C_{3-7}$-Cycloalkylcarbonyl, $C_{1-6}$-Alkoxycarbonyl, Phenoxycarbonyl, Furfuryl, Furoyl, N-[2-(3-indolyläthyl] oder N-(indol-3-yl-oxalyl),

n 1 oder 2

bedeuten,

sowie von deren pharmazeutisch unbedenklichen Säureadditionssalzen, dadurch gekennzeichnet, daß man

a) eine Verbindung der Formel

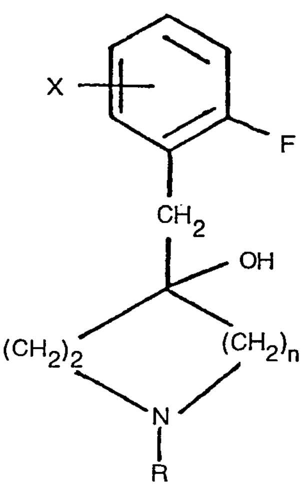

IV

in welcher X die oben angegebene Bedeutung hat und R $C_{1-6}$-Alkyl, $C_{2-6}$-Alkenyl, $C_{3-7}$-Cycloalkyl-$C_{1-6}$-alkyl oder Phenyl-$C_{1-6}$-alkyl bedeutet, in Gegenwart eines Lösungsmittels bei Temperaturen zwischen 25°C und der Rückflußtemperatur des Reaktionsgemisches mit einer nicht nucleophilen Base zu einer Verbindung der Formel I umsetzt, in der X und n die zur Formel I und R die zur Formel IV angegebenen Bedeutungen haben und

b) gegebenenfalls die so erhaltende Verbindung mit einem Chlorameisensäurealkylester oder einem Chlorameisensäurephenylester zu einer Verbindung der Formel I umsetzt, in welcher X und n die

oben angegebenen Bedeutungen haben und R $C_{1-6}$-Alkoxycarbonyl oder Phenoxycarbonyl bedeutet und

c) gegebenenfalls die so erhaltene Verbindung mit einer Base oder einer Säure zu einer Verbindung der Formel I umsetzt, in welcher X und n die oben angegebenen Bedeutungen haben und R Wasserstoff bedeutet,

d) gegebenenfalls eine Verbindung der Formel I, in welcher X und n die oben angegebene Bedeutung haben und R Benzyl bedeutet, zu einer Verbindung der Formel I, in welcher R Wasserstoff ist, hydriert und

e) gegebenenfalls eine Verbindung der Formel I, worin X und n die oben angegebenen Bedeutungen haben und R Wasserstoff ist, mit einem $C_{1-6}$-Alkanoylhalogenid oder -anhydrid, einem $C_{3-7}$-Cycloalkylcarbonylhalogenid, einem Phenyl-$C_{1-6}$-alkanoylhalogenid, einem Benzoylhalogenid oder -anhydrid, einem Furoylhalogenid oder -anhydrid oder einem $C_{1-6}$-Alkoxyoxalylhalogenid zu einer Verbindung der Formel I umsetzt, in welcher X und n die oben angegebenen Bedeutungen haben und R $C_{1-6}$-Alkanoyl, $C_{3-7}$-Cycloalkalcarbonyl, Phenyl-$C_{1-6}$-alkanoyl, Benzoyl, Furoyl oder $C_{1-6}$-Alkoxyoxalyl bedeutet,

f) gegebenenfalls eine Verbindung der Formel I, worin X und n die oben angegebenen Bedeutungen haben und R $C_{1-6}$-Alkanoyl, $C_{1-6}$-Alkoxyoxalyl, $C_{3-7}$-Cycloalkylcarbonyl, Phenyl-$C_{1-6}$-alkanoyl, Furoyl oder Indol-3-yloxalyl ist, zu einer Verbindung der Formel I reduziert, in welcher R $C_{1-6}$-Alkyl, Hydroxyäthyl, $C_{3-7}$-Cycloalkyl-$C_{1-6}$-alkyl, Phenyl-$C_{1-6}$-alkyl, Furfuryl oder 2-(Indol-3-yl)äthyl bedeutet,

g) gegebenenfalls eine Verbindung der Formel I, worin X und n die oben angegebenen Bedeutungen haben und R Wasserstoff ist, mit einer Verbindung der Formel R—Y, worin R $C_{1-6}$-Alkyl, $C_{2-6}$-Alkenyl, Phenyl-$C_{1-6}$-alkyl, Furfuryl, Indol-3-yloxalyl oder

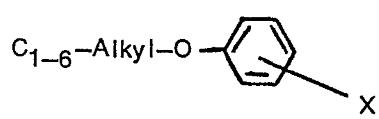

und Y ein Halogen bedeutet, zu einer Verbindung der Formel I umsetzt, in welcher R die oben angegebene Bedeutung hat,

h) gegebenenfalls eine Verbindung der Formel I, in der R $C_{1-6}$-Alkyl oder $C_{1-6}$-Alkanoyl bedeutet, n die oben angegebene Bedeutung hat und X Wasserstoff ist, nach üblichen Methoden nitriert zu einer Verbindung der Formel I, in welcher X die Nitro-Gruppe darstellt und

i) gegebenenfalls die so erhaltene Verbindung der Formel I zu einer Verbindung der Formel I, in der R $C_{1-6}$-Alkyl oder $C_{1-6}$-Alkanoyl bedeutet, n die oben angegebene Bedeutung hat und X die Amino-Gruppe ist, katalytisch reduziert und

k) gegebenenfalls eine Verbindung der Formel I, in welcher R $C_{1-6}$-Alkanoyl ist und X und n die oben angegebenen Bedeutungen haben, zu einer N-unsubstituierten Verbindung der Formel I hydrolysiert.

**Revendications**

1. Composés répondant à la formule:

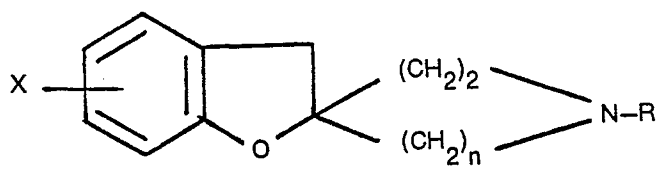

dans laquelle:
X désigne l'hydrogène, un groupe nitro, amino, chloro, fluoro, bromo ou méthoxy,
R désigne l'hydrogène, un groupe alcoyle en $C_{1-6}$, alcényle en $C_{2-6}$, hydroxyéthyle, alcanoyle en $C_{1-6}$, (alcoxy en $C_{1-6}$) oxalyle, un groupe phényl-(alcanoyle en $C_{1-6}$), benzoyle, phényl-(alcoyle en $C_{1-6}$) ou phénoxy(alcoyle en $C_{1-6}$) non substitué ou substitué, le radical phényle pouvant à chaque fois être substitué une ou deux fois par un groupe nitro, amino, chloro, fluoro, bromo ou méthoxy, un groupe (cycloalcoyl en $C_{3-7}$)-(alcoyle en $C_{1-6}$), (cycloalcoyle en $C_{3-7}$) carbonyle, (alcoxy en $C_{1-6}$)carbonyle, phénoxycarbonyle, furfuryle, furoyle, N-[2-(3-indolyléthyle)] ou N-(indol-3-yl-oxalyle),
n est égal à 1 ou 2,
ainsi que leurs sels d'addition avec des acides pharmaceutiquement acceptables.

2. 2,3-dihydro-1'-méthylspiro[benzofuranne-2,4'-pipéridine] ou ses sels d'addition avec des acides pharmaceutiquement acceptables.

3. 2,3-dihydro-1'-cyclobutylméthylspiro[benzofuranne-2,4'-pipéridine] ou ses sels d'addition avec des acides pharmaceutiquement acceptables.

4. 2,3-dihydro-1'-(β-phényléthyl)spiro[benzofuranne-2,4'-pipéridine] ou ses sels d'addition avec des acides pharmaceutiquement acceptables.

5. 2,3-dihydro-1'-(β-phénéthyl)spiro[benzofuranne-2,3'-pyrrolidine] ou ses sels d'addition avec des acides pharmaceutiquement acceptables.

6. 2,3-dihydro-5-nitrospiro[benzofuranne-2,4'-pipéridine] ou ses sels d'addition avec des acides pharmaceutiquement acceptables.

7. Composés répondant à la formule I, destinés à être untilisés comme médicaments.

8. Procédé de préparation de composés répondant à la formule I:

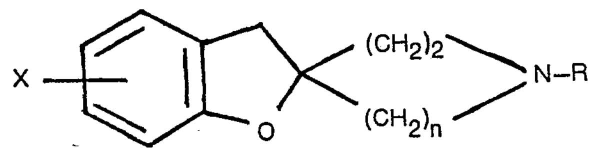

I

dans laquelle:
X désigne l'hydrogène, un groupe nitro, amino, chloro, fluoro, bromo ou méthoxy,
R désigné l'hydrogène, on groupe alcoyle en $C_{1-6}$, alcényle en $C_{2-6}$, hydroxyéthyle, alcanoyle en $C_{1-6}$, (alcoxy en $C_{1-6}$) oxalyle, phényl-(alcanoyle en $C_{1-6}$), benzoyle, phényl-(alcoyle en $C_{1-6}$) ou phénoxy-(alcoyle en $C_{1-6}$ substitué ou non substitué, le radical phényle pouvant à chaque fois être substitué une ou deux fois par un groupe nitro, amino, chloro, fluoro, bromo ou méthoxy, un groupe (cycloalcoyl en $C_{3-7}$)-(alcoyle en $C_{1-6}$), (cycloalcoyle en $C_{3-7}$) carbonyle, (alcoxy en $C_{1-6}$)-carbonyle, phénoxycarbonyle, furfuryle, furoyle, N-[2-(3-indolyléthyle)] ou N-(indol-3-yle-oxalyle),
n est égal à 1 ou 2,
ainsi que leurs sels d'addition avec des acides pharmaceutiquement acceptables, caractérisé en ce qu'on fait réagir

a) un composé répondant à la formule:

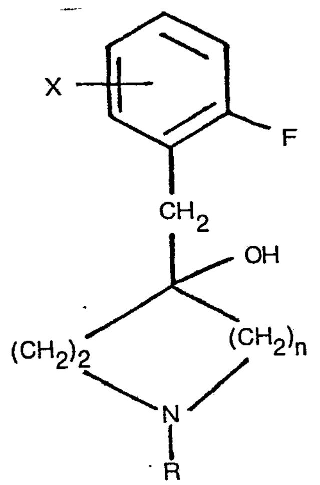

IV

dans laquelle X a la signification indiquée ci-dessus, et R désigne un groupe alcoyle en $C_{1-6}$, acényle en $C_{2-6}$, (cycloalcoyl en $C_{3-7}$)-(alcoyle en $C_{1-6}$), ou phényl-(alcoyle en $C_{1-6}$), en présence d'un solvant, à une température comprise entre 25°C et la température de reflux du mélange réactionnel, avec une base non nucléophile, pour donner un composé répondant à la formule I, dans laquelle X et n ont les significations indiquées à propos de la formule I, et R a la signification indiquée à propos de la formule IV, et

b) on fati réagir le cas échéant le composé ainsi obtenu avec un chloroformiate d'alcoyle ou avec un chloroformiate de phényle pour donner un composé répondant à la formule I, dans laquelle X et n ont les significations indiquées ci-dessus, et R désigne un groupe (alcoxy en $C_{1-6}$) carbonyle ou phénoxycarbonyle, et

c) on fait réagir le cas échéant le composé ainsi obtenu avec une base ou un acide pour donner un composé répondant à la formule I, dans laquelle X et n ont les significations indiquée ci-dessus, et R désigne l'hydrogène, et

d) on hydrogène le cas échéant un composé répondant à la formule I, dans laquelle X et n ont les significations indiquées ci-dessus, et R désigne un groupe benzyle, pour donner un composé répondant à la formule I, dans laquelle R est l'hydrogène et,

e) on fait réagir le cas échéant un composé répondant à la formule I, dans laquelle X et n ont les significations indiquées ci-dessus, et R est l'hydrogène, avec un halogénure ou un anhydride d'alcanoyle en $C_{1-6}$, un halogénure de (cycloalcoyl en $C_{3-7}$) carbonyle, un halogénure de phényl-(alcanoyle en $C_{1-6}$), un halogénure ou un anhydride de benzoyle, un halogénure ou un anhydride

de furoyle, ou un halogénure d'(alcoxy en $C_{1-6}$)oxalyle, pour donner un composé répondant à la formule I, dans laquelle X et n ont les significations indiquées ci-dessus, et R désigne un groupe alcanoyle en $C_{1-6}$, (cycloalcoyl en $C_{3-7}$)carbonyle, phényl-(alcanoyle en $C_{1-6}$), benzoyle, furoyle, ou (alcoxy en $C_{1-6}$)oxalyle,

f) on réduit les cas échéant un composé répondant à la formule I, dans laquelle X et n ont les significations indiquées ci-dessus, et R désigne un groupe alcanoyle en $C_{1-6}$, (alcoxy en $C_{1-6}$) oxalyle, (cycloalcoyle en $C_{3-7}$)-carbonyle, phényl-alcanoyle en $C_{1-6}$, furoyle ou indol-3-yloxalyle, pour donner un composé répondant à la formule I, dans laquelle R désigne un groupe alcoyle en $C_{1-6}$, un groupe hydroxyéthyle, un groupe (cycloalcoyl en $C_{3-7}$)-(alcoyle en $C_{1-6}$), un groupe phényl-(alcoyle en $C_{1-6}$), un groupe furfuryle ou un groupe 2-(indol-3-yl)éthyle,

g) on fait réagir le cas échéant un composé répondant à la formule I, dans laquelle X et n ont les significations indiquées ci-dessus, et R est l'hydrogène, avec un composé répondant à la formule R—Y, dans laquelle R désigne un groupe alcoyle en $C_{1-6}$, alcényle en $C_{2-6}$, phényle-(alcoyle en $C_{1-6}$), furfuryle indol-3-yloxalyle ou (alcoyl en

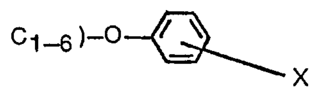

et Y désigne un halogène, pour donner un composé répondant à la formule I, dans laquelle R a la signification indiquée ci-dessus, et

h) on effectue la cas échéant une nitration d'un composé répondant à la formule I, dans laquelle R désigne un groupe alcoyle en $C_{1-6}$, ou alcanoyle en $C_{1-6}$, n a la signification indiquée ci-dessus, et X est l'hydrogène, par les procédés habituels, pour donner un composé répondant à la formule I, dans laquelle X représente le groupe nitro, et

i) on réduit catalytiquement le cas échéant le composé répondant à la formule I ainsi obtenu pour donner un composé répondant à la formule I dans laquelle R désigne un groupe alcoyle en $C_{1-6}$ ou alcanoyle en $C_{1-6}$, n a la signification indiquée ci-dessus, et X est le groupe amino, et

k) on hydrolyse le cas échéant un composé répondant à la formule I, dans laquelle R est un groupe alcanoyle en $C_{1-6}$, et X et n ont les significations indiquées ci-dessus, pour donner un composé N-non substitué répondant à la formule I.

## Claims

1. A compound of the formula

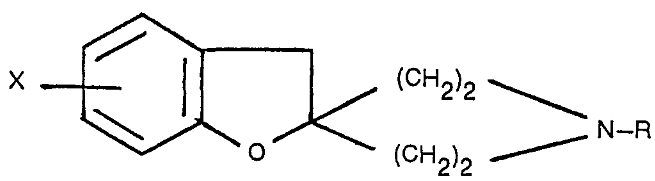

or a pharmaceutically acceptable acid addition salt thereof in which X is hydrogen, nitro, amino, chlorine, fluorine, bromine or methoxy; R is hydrogen, $C_{1-6}$-alkyl, $C_{2-6}$-alkenyl, hydroxyethyl, $C_{1-6}$-alkanoyl, $C_{1-6}$-alkoxyoxalyl, unsubstituted or substituted phenyl-$C_{1-6}$-alkanoyl, benzoyl, phenyl-$C_{1-6}$-alkyl, or phenoxy-$C_{1-6}$-alkyl wherein the phenyl may be substituted once or twice by nitro, amino, chlorine, fluorine, bromine or methoxy, $C_{3-7}$-cycloalkyl-$C_{1-6}$-alkyl, $C_{3-7}$-cycloalkylcarbonyl, $C_{1-6}$-alkoxycarbonyl, phenoxycarbonyl, furfuryl, furoyl, N-[2-(3-indolyl)ethyl] or N-(indol-3-yloxalyl); and n is the integer 1 or 2.

2. 2,3-Dihydro-1′-methylspiro[benzofuran-2,4′-piperidine] or a pharmaceutically acceptable acid addition salt thereof.

3. 2,3-Dihydro-1′-cyclobutylmethylspiro[benzofuran-2,4′-piperidine] or a pharmaceutically acceptable acid addition salt thereof.

4. 2,3-Dihydro-1′-(β-phenylethyl)spiro[benzofuran-2,4′-piperidine] or a pharmaceutically acceptable acid addition salt thereof.

5. 2,3-Dihydro-1′-(β-phenylethyl)spiro[benzofuran-2,3′-pyrrolidine] or a pharmaceutically acceptable acid addition salt thereof.

6. 2,3-Dihydro-5-nitrospiro[benzofuran-2,4′-piperidine] or a pharmaceutically acceptable acid addition salt thereof.

7. Compounds of the formula I for the use as medicaments.

8. A method of preparing a compound of the formula I

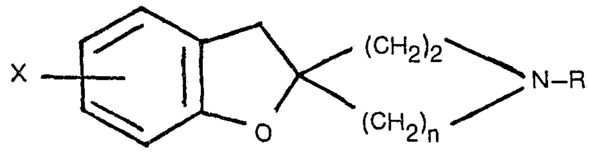

(I)

or a pharmaceutically acceptable acid addition salt thereof in which X is hydrogen, nitro, amino, chlorine, fluorine, bromine or methoxy; R is hydrogen, $C_{1-6}$-alkyl, $C_{2-6}$-alkenyl, hydroxyethyl, $C_{1-6}$-alkanoyl, $C_{1-6}$-alkoxyoxaly, unsubstituted or substituted phenyl-$C_{1-6}$-alkanoyl, benzoyl, phenyl-$C_{1-6}$-alkyl, or phenoxy-$C_{1-6}$-alkyl wherein the phenyl may be substituted once or twice by nitro, amino, chlorine, fluorine, bromine or methoxy, $C_{3-7}$-cycloalkyl-$C_{1-6}$-alkyl, $C_{3-7}$-cycloalkylcarbonyl, $C_{1-6}$-alkoxycarbonyl, phenoxycarbonyl, furfuryl, furoyl, N-[2-(3-indolyl)ethyl] or N-(indol-3-yloxalyl); and n is the integer 1 or 2 which comprises

a) reacting a compound of the formula

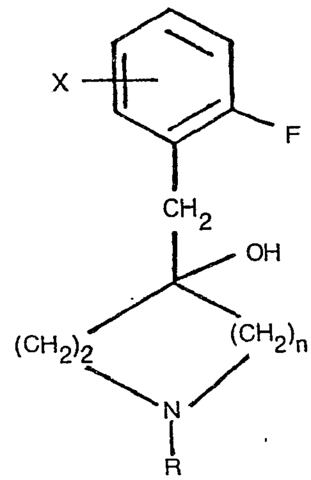

IV

in which X is as defined above and R is $C_{1-6}$-alkyl, $C_{2-6}$-alkenyl, $C_{3-7}$-cycloalkyl-$C_{1-6}$-alkyl or phenyl-$C_{1-6}$-alkyl with a non-nucleophilic base in the presence of a solvent at a temperature of from 25°C to the reflux point of the reaction mixture, to provide a compound of the formula I in which X and n are as defined above and R is as defined in formula IV above, and

b) optionally treating the so-obtained compound with an alkylchloroformate or a phenylchloroformate to provide a compound of the formula I in which X and n are as defined above and R is $C_{1-6}$-alkoxycarbonyl or phenoxycarbonyl and,

c) optionally reacting the so-obtained compound with a base or an acid to provide a compound of the formula I in which X and n are as defined above and R is hydrogen,

d) optionally hydrogenating a compound of the formula I in which X and n are as defined above and R is benzyl to provide a compound of the formula I in which R is hydrogen, and

e) optionally reacting a compound of the formula I in which X and n are as defined above, and R is hydrogen, with a $C_{1-6}$-alkanoyl-halide or -anhydride, $C_{3-7}$-cycloalkylcarbonyl-halide, phenyl-$C_{1-6}$-alkanoyl halide, benzoyl-halide or -anhydride, furoyl-halide or -anhydride or $C_{1-6}$-alkoxyoxalyl halide to provide a compound of the formula I in which x and n are as defined above and R is $C_{1-6}$-alkanoyl, $C_{3-7}$-cyclocarbonyl, phenyl-$C_{1-6}$-alkanoyl, benzoyl, furoyl or $C_{1-6}$-alkoxyoxalyl, and

f) optionally reducing a compound of the formula I in which X and n are as defined as above and R is $C_{1-6}$-alkanoyl, $C_{1-6}$-alkoxyoxalyl, $C_{3-7}$-cycloalkylcarbonyl, phenyl-$C_{1-6}$-alkanoyl, furoyl or indol-3-yloxalyl to provide a compound of the formula I in which R is $C_{1-6}$-alkyl, hydroxyethyl, $C_{3-7}$-cycloalkyl-$C_{1-6}$-alkyl, phenyl-$C_{1-6}$-alkyl, furfuryl or 2-(indol-3-yl)ethyl, or

g) optionally reacting a compound of the formula I in which X and n are as defined above and R is hydrogen with a compound of the formula R—Y in which R is $C_{1-6}$-alkyl, $C_{2-6}$-alkenyl, phenyl-$C_{1-6}$-alkyl, furfuryl, indol-3-yloxalyl or

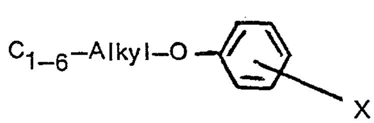

and Y is a halogen to provide a compound of the formula I in which R is as defined above, and

h) optionally treating a compound of the formula I in which R is $C_{1-6}$-alkyl or $C_{1-6}$-alkanoyl, n is as defined above and x is hydrogen according to usual methods to provide a compound of the

formula I in which X is the nitro group and

i) optionally catalytically reducing the so-obtained compound of the formula I to provide a compound of the formula I in which R is $C_{1-6}$-alkyl or $C_{1-6}$-alkanoyl, n is as defined above and X is the amino group, and

k) optionally subjecting a compound of the formula I in which R is $C_{1-6}$-alkanoyl and X and n are as defined above to hydrolysis to provide an N-unsubstituted compound of the formula I.